# EUROPEAN PATENT APPLICATION

(11) **EP 4 603 481 A1**
(43) Date of publication of application: **20.08.2025**
(21) Application number: 23905881.1
(22) Date of filing: 18.12.2023
(51) Int. Cl.: C07D 295/13, A61K 31/40, A61K 31/495, A61K 9/127, A61P 35/00

(54) **PREPARATION AND USE OF CATIONIC LIPID MATERIAL**

(30) Priority: 21.12.2022 CN 202211652875
(71) Applicant: Nanjing Geneleap Biotechnology Co., Ltd., Nanjing, Jiangsu 210016 (CN); Nanjing Luye Pharmaceutical Co., Ltd., Jiangsu 210061 (CN)
(72) Inventor: WANG, Jinhai, Nanjing, Jiangsu 210016 (CN); CHENG, Xinwei, Nanjing, Jiangsu 210016 (CN); LIU, Ning, Nanjing, Jiangsu 210016 (CN); LI, Xiaoji, Nanjing, Jiangsu 210016 (CN); ZHENG, Yifei, Nanjing, Jiangsu 210016 (CN)
(74) Representative: karo IP
(86) International application number: PCT/CN2023/139463
(87) International publication number: WO 2024/131717

(57) **Abstract**

The present invention relates to the field of biomedicines, in particular to cationic lipid compounds capable of being used for nucleic acid delivery, a preparation method therefor and the use thereof. LNPs prepared from the cationic lipid compounds with novel structures have the advantages of high transfection efficiency, etc., and in particular, the LNPs are distributed only at injection sites.

## Description

### TECHNICAL FIELD

The present invention relates to the field of biomedicines, particularly to compounds that can be used for nucleic acid delivery, and their preparation methods and uses.

### BACKGROUND ART

Recently, with the global outbreak of COVID-19, remarkable success has been made in COVID-19 mRNA vaccines. Currently, the three mRNA vaccine giants, Modema, CureVac and BioNTech, all use Lipid NanoParticles (LNP) delivery technology for their COVID-19 vaccines. The cationic lipid material in the LNP delivery technology plays a critical role. Nucleic acid drugs need to be encapsulated into nanoparticles by specific microfluidic technology, which protects mRNA and facilitates the delivery across cell membranes. In addition, it promotes the endosomal escape and the release of mRNA drugs. LNP preparations play a vital role in the field of nucleic acid drug delivery.

At present, the commonly used cationic lipid materials include non-ionized cationic materials and ionizable cationic materials.

As for non-ionized cationic materials, the LNPs made from this type of lipid materials take advantage of their electropositivity to make its relatively stable and alsoimprove the transfection efficiency and overall expression at cellular level. However, they also face several challenges: (1) The LNP made from a non-ionized cationic material has a common issue of systemic toxicity in vivo, which affects its dosage and corresponding efficacy; (2) The delivery efficiency of the LNP made from a non-ionized cationic lipids is generally low in vivo, which makes it limited use in clinical practice..

Further, as for ionizable cationic materials, plenty of COVID-19 vaccine products using the LNP delivery technology have been launched in the market at present, but they encountered a few challenges: (1) The structures of cationic materials *per se* are protected by big pharmas, and it is difficult to make structural innovation; (2) LNP composition ratio is also under patent protection, and the LNP prepared using same composition ratio of commercially available products have the risk of infringement; (3) As a vaccine product, the commercially available product shows strong accumulation in the liver and may have some safety issues.

In summary, in view of the above problems and challenges, in order to overcome problems such as in vivo toxicity and delivery efficiency, it is still necessary to have novel design and synthesizenew cationic lipid materials, and fully use LNP delivery technique to achieve efficient drug delivery.

### SUMMARY OF THE INVENTION

In order to meet the urgent clinical need of nucleic acid drugs delivery for different diseases, and to reduce systemic toxicity, lower the liver accumulation risk and improve delivery efficiency, the objective of the present invention is to provide novel a series of cationic lipid compounds and its preparation method and application. The LNP made from a cationic lipid compound with a new structure in the present invention has several advantages such as high transfection efficiency. For example, LNP containing a non-ionized cationic lipid compound 3 is mainly distributed at the injection site and rarely enters the system through blood circulation. For LNPs containing ionizable cationic lipids compound 1 and compound 2, they are mainly accumulated at the injection site at 25 mol% ratio compared with MC3 formulation; the in vivo transfection efficiency of the LNP containing compound 2 at 50 mol% prescription ratio is equivalent to that of MC3, and the in vivo transfection efficiency of the LNP containing compound 1 at 50 mol% prescription ratio is 1.668 times that of MC3. Further, the LNPs containing ionizable cationic lipids compound 17 and compound 18 completely avoid liver toxicity while achieving local transfection equivalent to that of MC3 LNP.

The present invention provides a cationic lipid compound as shown in Formula (I) or a pharmaceutically acceptable salt thereof: 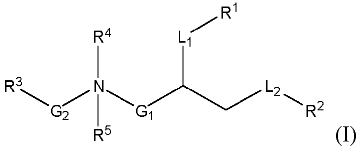 where,
R¹ and R² each independently are C₆-C₂₀ alkyl or C₆-C₂₀ alkenyl, or substituted C₂-C₅ alkyl, in which the substituent is -OC(=O)-, -C(=O)O-, -SC(=O)- or -C(=O)S- substituted by C₁₆-C₂₀ alkyl;
G₁ and G₂ each independently are C₁-C₄ alkylene;
L₁ and L₂ each independently are -OC(=O)-, -C(=O)O-, -SC(=O)- or -C(=O)S-;
R³ independently is a 5-6 membered saturated heterocyclic group containing 1 or 2 heterocyclic atoms independently selected from N, O and S and optionally substituted by R⁶;
R⁴ independently is C₁-C₄ alkyl, -OH or -SH, of which alkyl is optionally substituted by -OH or -SH;
R⁵ independently is absent or C₁-C₄ alkyl;
R⁶ independently is C₁-C₆ alkyl, which is optionally substituted by -OH or -SH.

In an embodiment of the present invention, in the lipid compound shown in the above formula (I), R¹ and R² each independently are C₆-C₂₀ alkyl or C₆-C₂₀ alkenyl, preferably C₁₀-C₂₀ alkyl or C₁₀-C₂₀ alkenyl, more preferably C₁₅-C₂₀ alkyl or C₁₅-C₂₀ alkenyl.

In an embodiment of the present invention, in the lipid compound shown in the above formula (I), R¹ and R² each independently are 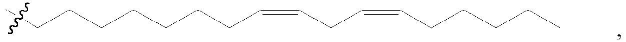 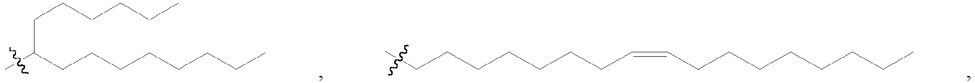 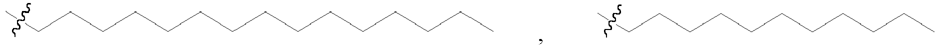 or

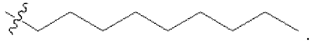

In an embodiment of the present invention, in the lipid compound shown in the above formula (I), R¹ and R² each independently are substituted C₂-C₅ alkyl, preferably substituted C₄ alkyl, in which the substituent is -OC(=O)-, -C(=O)O-, -SC(=O)- or -C(=O)S- substituted by C₁₆-C₂₀ alkyl.

In an embodiment of the present invention, in the lipid compound shown in the above formula (I), R¹ and R² each independently are

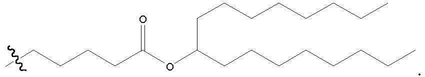

In an embodiment of the present invention, in the lipid compound shown in the above formula (I), G₁ independently is methylene, and G₂ independently is methylene or ethylene.

In an embodiment of the present invention, in the lipid compound shown in the above formula (I), R³ independently is pyrrolidinyl or piperazinyl, which is optionally substituted by R⁶, and R⁶ independently is methyl, ethyl, propyl or butyl, which is optionally substituted by -OH or -SH.

In an embodiment of the present invention, in the lipid compound shown in the above formula (I), R⁴ independently is methyl, hydroxymethyl, hydroxyethyl or -OH, and R⁵ is absent.

In an embodiment of the present invention, in the lipid compound shown in the above formula (I), R⁴ independently is methyl, and R⁵ independently is methyl.

The present invention provides a cationic lipid compound shown in Formula (I) or a pharmaceutically acceptable salt thereof: 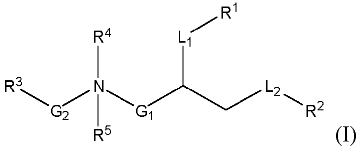 where,
R¹ and R² each independently are C₆-C₂₀ alkyl or C₆-C₂₀ alkenyl;
G₁ and G₂ each independently are C₁-C₄ alkylene;
L₁ and L₂ each independently are -OC(=O)-, -C(=O)O-, -SC(=O)- or -C(=O)S-;
R³ independently is a 5-6 membered saturated heterocyclic group containing 1 or 2 heterocyclic atoms independently selected from N, O and S and optionally substituted by R⁶;
R⁴ independently is C₁-C₄ alkyl, -OH or -SH, of which alkyl is optionally substituted by -OH or -SH;
R⁵ independently is absent or C₁-C₄ alkyl;
R⁶ independently is C₁-C₆ alkyl, which is optionally substituted by -OH or -SH.

In an embodiment of the present invention, in the lipid compound shown in the above formula (I), R¹ and R² each independently are C₁₀-C₂₀ alkyl or C₁₀-C₂₀ alkenyl, preferably C₁₅-C₂₀ alkyl or C₁₅-C₂₀ alkenyl.

In an embodiment of the present invention, in the lipid compound shown in the above formula (I), R¹ and R² each independently are 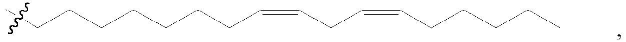 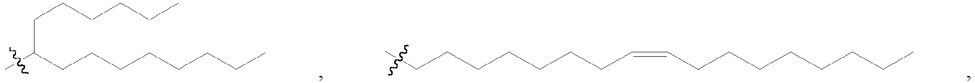 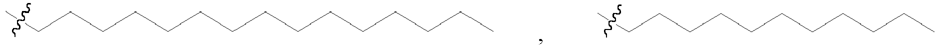 or

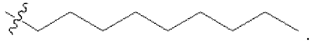

In an embodiment of the present invention, in the lipid compound shown in the above formula (I), G₁ independently is methylene, and G₂ independently is methylene or ethylene.

In an embodiment of the present invention, in the lipid compound shown in the above formula (I), R³ independently is pyrrolidinyl or piperazinyl, which is optionally substituted by R⁶, and R⁶ independently is methyl, ethyl, propyl or butyl, which is optionally substituted by -OH or -SH.

In an embodiment of the present invention, in the lipid compound shown in the above formula (I), R⁴ independently is methyl, hydroxymethyl, hydroxyethyl or -OH, and R⁵ is absent.

In an embodiment of the present invention, in the lipid compound shown in the above formula (I), R⁴ independently is methyl, and R⁵ independently is methyl.

The present invention provides an ionizable cationic lipid compound shown in Formula (Ia) or a pharmaceutically acceptable salt thereof: 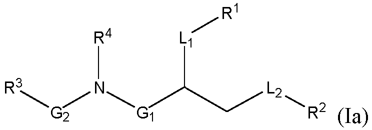 where,
R¹ and R² each independently are C₆-C₂₀ alkyl or C₆-C₂₀ alkenyl, or substituted C₂-C₅ alkyl, in which the substituent is -OC(=O)-, -C(=O)O-, -SC(=O)- or -C(=O)S- substituted by C₁₆-C₂₀ alkyl;
G₁ and G₂ each independently are C₁-C₄ alkylene;
L₁ and L₂ each independently are -OC(=O)-, -C(=O)O-, -SC(=O)- or -C(=O)S-;
R³ independently is a 5-6 membered saturated heterocyclic group containing 1 or 2 heterocyclic atoms independently selected from N, O and S and optionally substituted by R⁶;
R⁴ independently is C₁-C₄ alkyl, -OH or -SH, of which the alkyl is optionally substituted by -OH or -SH;
R⁶ independently is C₁-C₆ alkyl, which is optionally substituted by -OH or -SH.

In an embodiment of the present invention, in the lipid compound shown in the above Formula (Ia), R¹ and R² each independently are C₆-C₂₀ alkyl or C₆-C₂₀ alkenyl, preferably C₁₀-C₂₀ alkyl or C₁₀-C₂₀ alkenyl, more preferably C₁₅-C₂₀ alkyl or C₁₅-C₂₀ alkenyl.

In an embodiment of the present invention, in the lipid compound shown in the above Formula (Ia), R¹ and R² each independently are 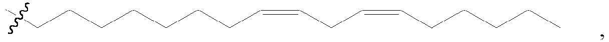 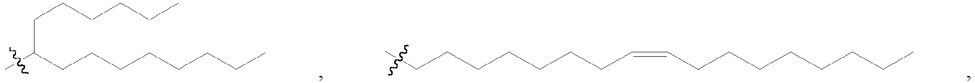 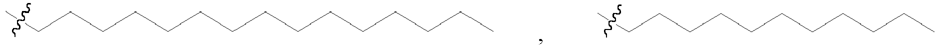 or

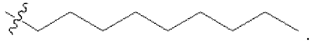

In an embodiment of the present invention, in the lipid compound shown in the above Formula (Ia), R¹ and R² each independently are substituted C₂-C₅ alkyl, preferably substituted C₄ alkyl, in which the substituent is -OC(=O)-, -C(=O)O-, -SC(=O)- or -C(=O)S- substituted by C₁₆-C₂₀ alkyl.

In an embodiment of the present invention, in the lipid compound shown in the above Formula (Ia), R¹ and R² each independently are

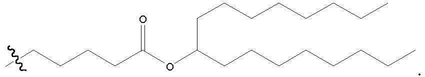

In an embodiment of the present invention, in the lipid compound shown in the above Formula (Ia), G₁ independently is methylene, and G₂ independently is methylene or ethylene.

In an embodiment of the present invention, in the lipid compound shown in the above Formula (Ia), R³ independently is pyrrolidinyl or piperazinyl, which is optionally substituted by R⁶, and R⁶ independently is methyl, ethyl, propyl or butyl, which is optionally substituted by -OH or -SH.

In an embodiment of the present invention, in the lipid compound shown in the above Formula (Ia), R⁴ independently is methyl, hydroxymethyl, hydroxyethyl or -OH.

The present invention provides an ionizable cationic lipid compound shown in Formula (Ia) or a pharmaceutically acceptable salt thereof: 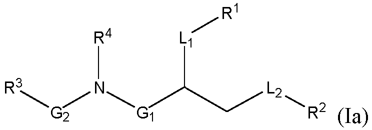 where,
R¹ and R² each independently are C₆-C₂₀ alkyl or C₆-C₂₀ alkenyl;
G₁ and G₂ each independently are C₁-C₄ alkylene;
L₁ and L₂ each independently are -OC(=O)-, -C(=O)O-, -SC(=O)- or -C(=O)S-;
R³ independently is a 5-6 membered saturated heterocyclic group containing 1 or 2 heterocyclic atoms independently selected from N, O and S and optionally substituted by R⁶;
R⁴ independently is C₁-C₄ alkyl, -OH or -SH, of which the alkyl is optionally substituted by -OH or -SH;
R⁶ independently is C₁-C₆ alkyl, which is optionally substituted by -OH or -SH.

In an embodiment of the present invention, in the lipid compound shown in the above Formula (Ia), R¹ and R² each independently are C₁₀-C₂₀ alkyl or C₁₀-C₂₀ alkenyl, preferably C₁₅-C₂₀ alkyl or C₁₅-C₂₀ alkenyl.

In an embodiment of the present invention, in the lipid compound shown in the above Formula (Ia), R¹ and R² each independently are 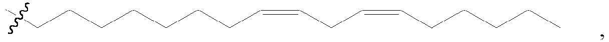 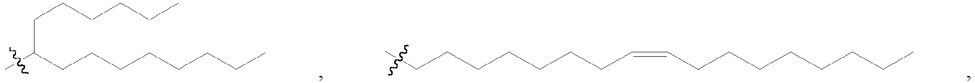 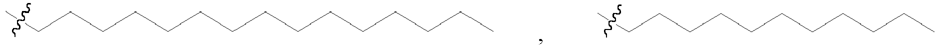 or

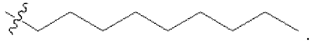

In an embodiment of the present invention, in the lipid compound shown in the above Formula (Ia), G₁ independently is methylene, and G₂ independently is methylene or ethylene.

In an embodiment of the present invention, in the lipid compound shown in the above Formula (Ia), R³ independently is pyrrolidinyl or piperazinyl, which is optionally substituted by R⁶, and R⁶ independently is methyl, ethyl, propyl or butyl, which is optionally substituted by -OH or -SH.

In an embodiment of the present invention, in the lipid compound shown in the above Formula (Ia), R⁴ independently is methyl, hydroxymethyl, hydroxyethyl or -OH.

The present invention provides a non-ionized cationic lipid compound shown in Formula (Ib) or a pharmaceutically acceptable salt thereof: 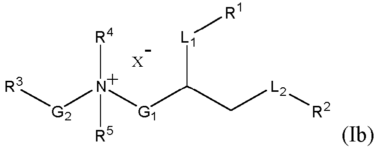 where,
R¹ and R² each independently are C₆-C₂₀ alkyl or C₆-C₂₀ alkenyl;
G₁ and G₂ each independently are C₁-C₄ alkylene;
L₁ and L₂ each independently are -OC(=O)-, -C(=O)O-, -SC(=O)- or -C(=O)S-;
R³ independently is a 5-6 membered saturated heterocyclic group containing 1 or 2 heterocyclic atoms independently selected from N, O and S and optionally substituted by R⁶;
R⁴ independently is C₁-C₄ alkyl;
R⁵ independently is C₁-C₄ alkyl;
R⁶ independently is C₁-C₆ alkyl, which is optionally substituted by -OH or -SH;
X is a chlorine, bromine or iodine atom.

In an embodiment of the present invention, in the lipid compound shown in the above Formula (Ib), R¹ and R² each independently are C₁₀-C₂₀ alkyl or C₁₀-C₂₀ alkenyl, preferably C₁₅-C₂₀ alkyl or C₁₅-C₂₀ alkenyl.

In an embodiment of the present invention, in the lipid compound shown in the above Formula (Ib), R¹ and R² each independently are 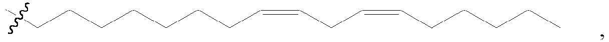 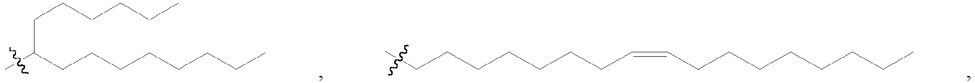 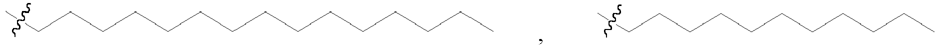 or

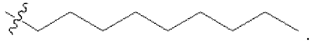

In an embodiment of the present invention, in the lipid compound shown in the above Formula (Ib), G₁ independently is methylene, and G₂ independently is methylene or ethylene.

In an embodiment of the present invention, in the lipid compound shown in the above Formula (Ib), R³ independently is pyrrolidinyl or piperazinyl, which is optionally substituted by R⁶, and R⁶ independently is methyl, ethyl, propyl or butyl, which is optionally substituted by -OH or -SH.

In an embodiment of the present invention, in the lipid compound shown in the above Formula (Ib), R⁴ independently is methyl, and R⁵ independently is methyl.

In an embodiment of the present invention, in the lipid compound shown in the above Formula (I), (Ia) or (Ib), the compound is selected from:

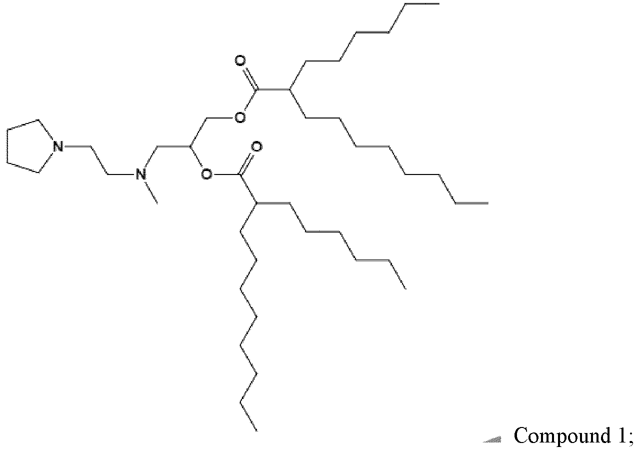

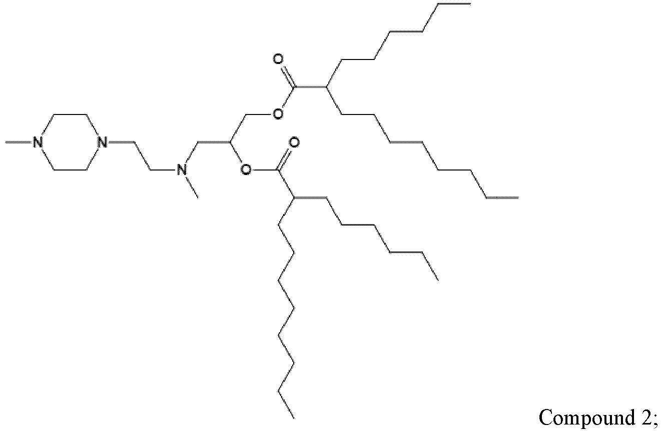

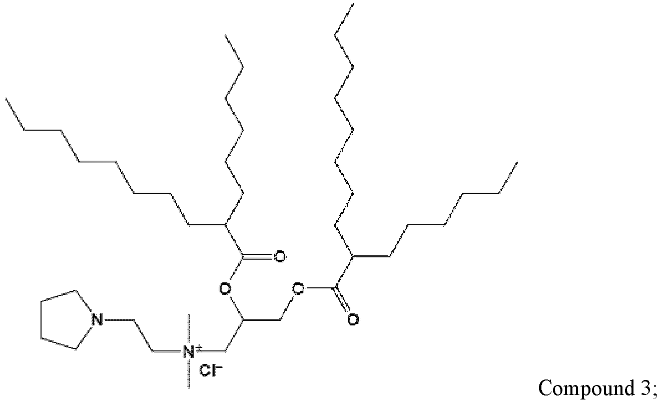

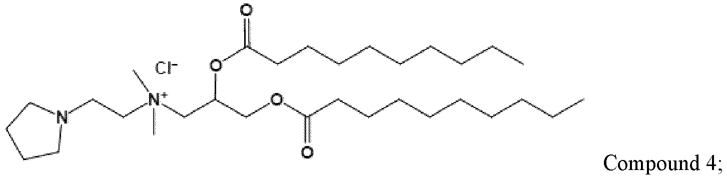

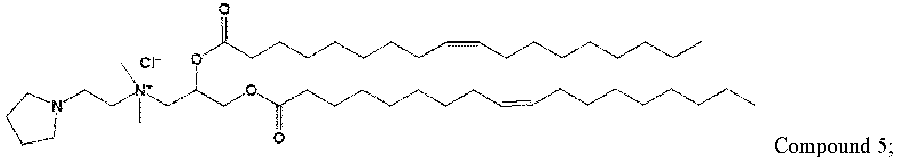

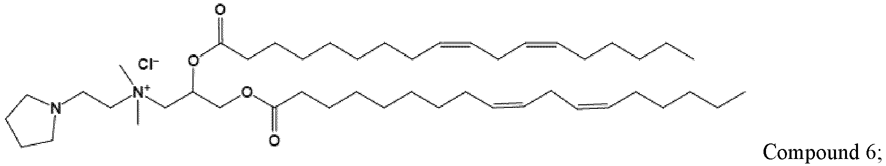

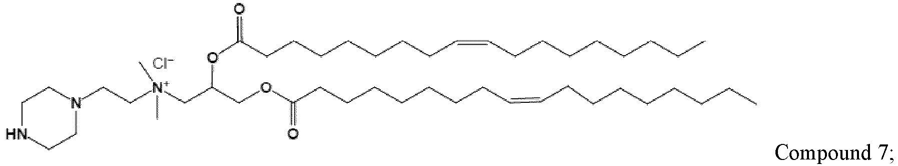

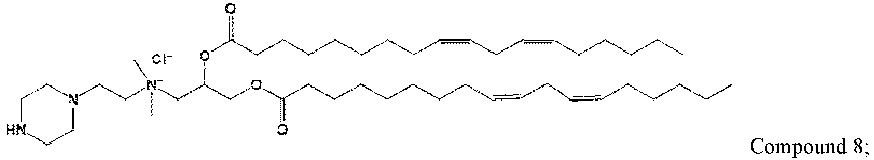

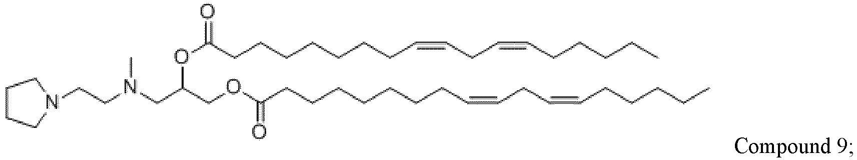

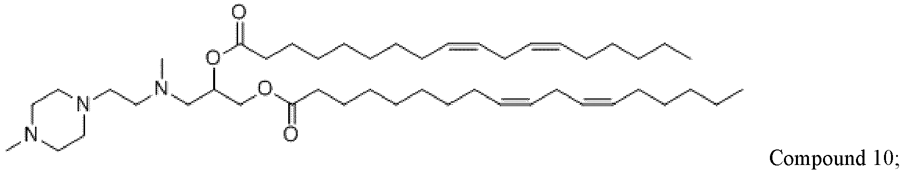

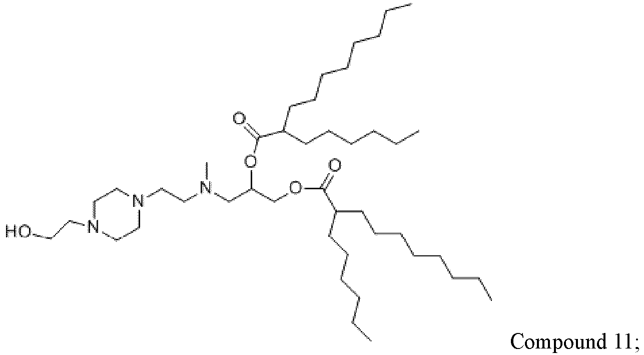

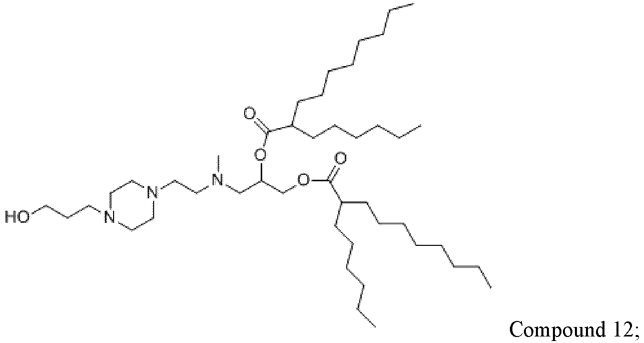

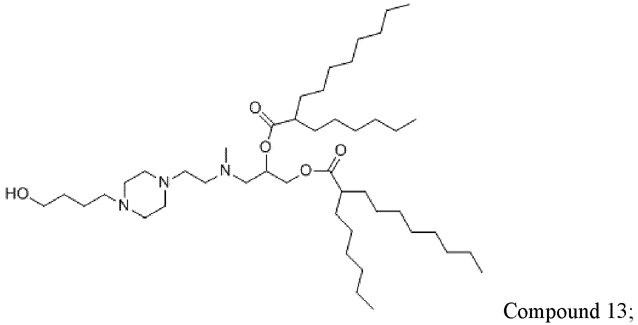

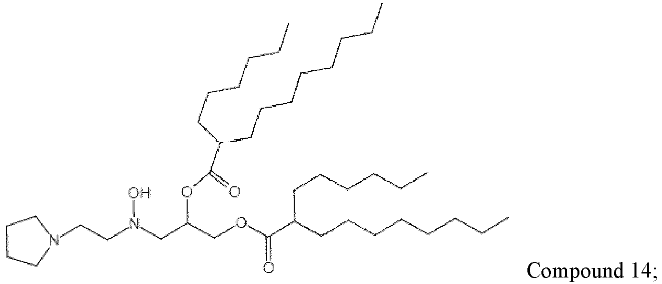

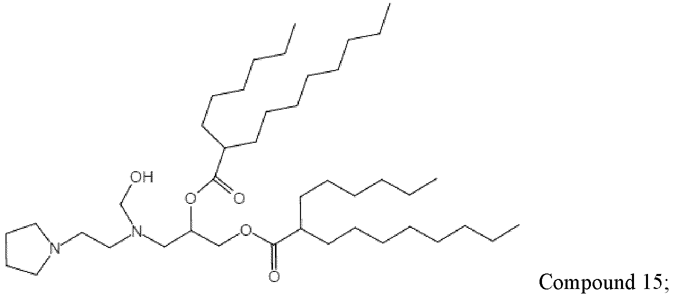

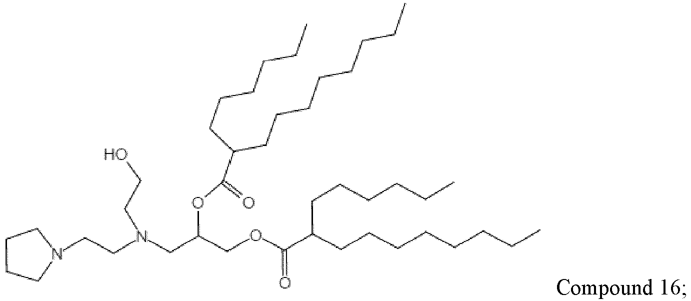

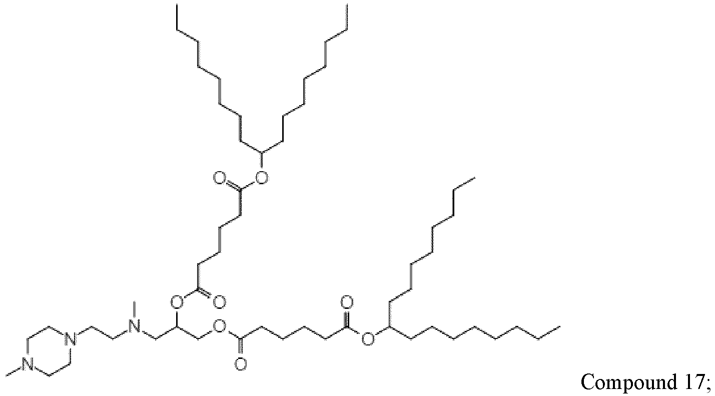

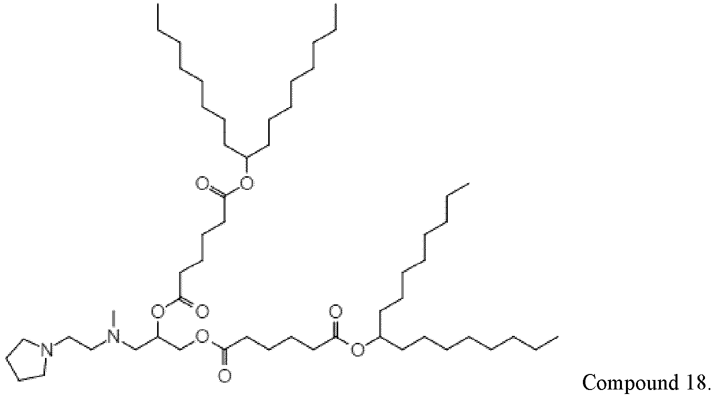

The present invention further provides a lipid nanoparticle composition, which contains a cationic lipid compound shown in the above Formula (I), (Ia) or (Ib) in the present application or a pharmaceutically acceptable salt thereof.

In an embodiment of the present invention, the foregoing lipid nanoparticle composition further contains neutral lipid, cholesterol and PEG lipid.

In an embodiment of the present invention, in the foregoing lipid nanoparticle composition, the neutral lipid is selected from DSPC, DOPC, DPPC, DOPG, DPPG, DOPE, POPC, POPE, DOPE-mal, DPPE, DMPE, DSPE, SOPE and 1,2-divaleryl-sn-glycerol-3-phosphoethanolamine (trans-DOPE), and the PEG lipid is selected from PEG-DMG, PEG-dipalmitoyl glycerol, PEG-DSPE, PEG-dilauryl glyceramide, PEG-dimyristyl glyceramide, PEG-dipalmitoyl glyceramide and PEG-distearoyl glyceramide, PEG-cholesterol (1-[8'-(cholest-5-ene-3[β]-oxo)fonnamido-3',6'-dioxazoctyl]carbamoyl-[ω]-methyl-poly(ethylene glycol), PEG-DMB, mPEG-DMG-2k, PEG2k-DMG, PEG2k-DSPE, PEG2k-DSG, PEG2k-DMA and PEG2k-DSA.

In an embodiment of the present invention, in the foregoing lipid nanoparticle composition, the neutral lipid is DSPC or DOPE, and the PEG lipid is mPEG-DMG-2k.

In an embodiment of the present invention, in the foregoing lipid nanoparticle composition, the molar percentage (mol %) of the cationic lipid compound described in the present application to the total lipid components in the composition is 15-60%.

In an embodiment of the present invention, in the foregoing lipid nanoparticle composition, the molar percentage (mol %) of the cationic lipid compound described in the present application to the total lipid components in the composition is 25-60%.

In an embodiment of the present invention, in the foregoing lipid nanoparticle composition, the molar percentage (mol %) of the cationic lipid compound described in the present application to the total lipid components in the composition is 25-50%.

In an embodiment of the present invention, in the foregoing lipid nanoparticle composition, the molar percentage (mol %) of the cationic lipid compound described in the present application to the total lipid components in the composition is 30-50%.

In an embodiment of the present invention, in the foregoing lipid nanoparticle composition, the molar percentage (mol %) of the cationic lipid compound described in the present application to the total lipid components in the composition is 40-50%.

In an embodiment of the present invention, in the foregoing lipid nanoparticle composition, the molar percentage of each of the lipid components described in the present application to the total lipid components of the composition is as follows, respectively: the cationic lipid compound 15-60%, the neutral lipid 5-50%, the cholesterol 30-56% and the PEG lipid 1.5-2.5%, and the sum of the molar percentages of the foregoing components is 100%.

In an embodiment of the present invention, in the foregoing lipid nanoparticle composition, the molar percentage of each of the lipid components described in the present application to the total lipid components of the composition is as follows, respectively: the cationic lipid compound 25-60%, the neutral lipid 10-45%, the cholesterol 30-56% and the PEG lipid 1.5-2.5%, and the sum of the molar percentages of the foregoing components is 100%.

In an embodiment of the present invention, in the foregoing lipid nanoparticle composition, the molar percentage of each of the lipid components described in the present application to the total lipid components of the composition is as follows, respectively: the cationic lipid compound 25%, the neutral lipid 43.5%, the cholesterol 30% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 50%, the neutral lipid 10%, the cholesterol 38.5% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 31.5%, the neutral lipid 10%, the cholesterol 56% and the PEG lipid 2.5%.

In an embodiment of the present invention, in the foregoing lipid nanoparticle composition, the molar percentage of each of the lipid components described in the present application to the total lipid components of the composition is as follows, respectively: the cationic lipid compound 43.3%, the neutral lipid 8.7%, the cholesterol 46.5% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 46.3%, the neutral lipid 9.5%, the cholesterol 42.7% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 50%, the neutral lipid 18.5%, the cholesterol 30% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 57.1%, the neutral lipid 7.1%, the cholesterol 34.3% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 15%, the neutral lipid 48.5%, the cholesterol 35% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 45%, the neutral lipid 10%, the cholesterol 43.5% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 45%, the neutral lipid 15%, the cholesterol 38.5% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 45%, the neutral lipid 18.5%, the cholesterol 35% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 45%, the neutral lipid 22.5%, the cholesterol 31% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 40%, the neutral lipid 12.5%, the cholesterol 46% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 40%, the neutral lipid 15%, the cholesterol 43.5% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 40%, the neutral lipid 20%, the cholesterol 38.5% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 36.5%, the neutral lipid 16%, the cholesterol 46% and the PEG lipid 1.5%.

In an embodiment of the present invention, in the foregoing lipid nanoparticle composition, the molar percentage of each of the lipid components described in the present application to the total lipid components of the composition is as follows, respectively: the cationic lipid compound 50%, the neutral lipid 10%, the cholesterol 38.5% and the PEG lipid 1.5%.

In an embodiment of the present invention, the foregoing lipid nanoparticle composition further contains a nucleic acid molecule, which is selected from mRNA, siRNA, antisense oligonucleotide (ASO), saRNA, miRNA and DNA. Specifically, the mRNA is selected from FFluc mRNA (SEQ ID NO.1) and HPV mRNA (SEQ ID NO.2).

In an embodiment of the present invention, in the foregoing lipid nanoparticle composition, the mass ratio of the total lipid components to the nucleic molecules in the composition is 15:1-40:1, preferably 20:1 or 40:1.

The present invention further provides a method for preparing the foregoing lipid nanoparticle composition, comprising: adding the prepared cationic lipid compound described in the present application, DSPC, cholesterol and mPEG-DMG-2k into an ethanol solution at a specific ratio, which is the ratio of the molar percentages of each of the lipid components described in the present application to the total lipid components of the composition, at a total lipid concentration of 23 mg/ml, and mixing them thoroughly; diluting mRNA with a malic acid or sodium acetate buffer (pH=3 or 4) to 0.2-0.4 mg/ml; mixing the foregoing lipid ethanol solution with the mRNA buffer aqueous solution on a microfluidic device at a volume ratio of 1:3-1:5, a flow rate of 10-15 ml/min and a mixing temperature of 50-60°C, and purifying the obtained lipid nanoparticles by the ultrafiltration or dialysis method to obtain ultimate lipid nanoparticles.

In an embodiment of the present invention, in the foregoing method for preparing the lipid nanoparticle composition, the volume ratio of the lipid ethanol solution to the mRNA aqueous solution is 1:3, the flow rate is 10 mL/min, and the mixing temperature is 50°C.

The present invention further provides a method for delivering nucleic acids into cells, comprising delivering the foregoing lipid nanoparticles as described in the present application into the cells.

In an embodiment of the present invention, the cells are mammalian cells, preferably human cells.

The present invention also provides a use of the foregoing lipid nanoparticles as described in the present application in the manufacture of a medicament or vaccine for the treatment of diseases, preferably, the disease is cancer, tuberculosis, helicobacter pylori, human papillomavirus (HPV), herpes zoster or dyslipidemia.

Unless otherwise stated, the following terms and phrases used herein are intended to have the following meanings. A specific term or phrase should not be considered uncertain or unclear in the absence of a specific definition, but should be understood in its ordinary meaning. When a trade name appears in this document, it is intended to refer to its corresponding product or its active ingredient.

The term "pharmaceutically acceptable" as used in the present application refers to compounds, materials, compositions and/or dosage forms that are within the scope of reliable medical judgment, suitable for use in contact with human and animal tissues, without excessive toxicity, irritation, anaphylaxis, or other problems or complications, and commensurate with a reasonable benefit/risk ratio.

As used in the present application, the term "pharmaceutically acceptable salt" means a salt of the compound provided by the present invention, which is made from a compound with a specific substituent discovered by the present invention and a relatively non-toxic acid or alkali. When the compound provided by the present invention contains a relatively acidic functional group, an alkali addition salt can be obtained by contacting a neutral form of such compound with a sufficient amount of alkali in a pure solution or an appropriate inert solvent. Examples of pharmaceutically acceptable acid addition salts include inorganic salts, organic salts, salts of amino acids (such as arginine), and salts of organic acids such as glucuronic acid (see Berge et al., "Pharmaceutical Salts", Journal of Pharmaceutical Science 66: 1-19 (1977)). Certain specific compounds provided by the present invention contain both alkaline and acidic functional groups, which can be converted into either alkali or acid addition salts.

The pharmaceutically acceptable salts in the present invention can be synthesized by conventional chemical methods from parent compounds containing acidic or basic groups. Generally, the salts are prepared by the following method: the salts are prepared from the reaction between these compounds in the form of free acid or alkali and stoichiometric appropriate alkalis or acids in water or an organic solvent or a mixture thereof.

The term "alkyl" or "alkylene" adopted in the present application is intended to include a saturated aliphatic hydrocarbon group with branched and straight chains having a specified number of carbon atoms. For example, "C₁-C₁₀ alkyl (or alkylene)" is intended to include C₁, C₂, C₃, C₄, C₅, C₆, C₇, C₈, C₉ and C₁₀ alkyl (or alkylene). Alkyl can be unsubstituted or substituted, in which at least one hydrogen is replaced by another chemical group. Examples of alkyl include without limitation methyl (Me), ethyl (Et), propyl (e.g., n-propyl and iso-propyl), butyl (e.g., n-butyl, iso-butyl, tert-butyl) and amyl (e.g., n-amyl, iso-amyl, neo-pentyl). Examples of alkylene include without limitation methylene, ethylene, propylidene and butylidene.

Specifically, the term "C₆-C₂₀ alkyl" adopted in the present application includes C₆, C₇, C₈, C₉, C₁₀, C₁₁, C₁₂, C₁₃, C₁₄, C₁₅, C₁₆, C₁₇, C₁₈, C₁₉ and C₂₀ alkyl; preferably, the term "C₁₀-C₂₀ alkyl" adopted in the present application includes C₁₀, C₁₁, C₁₂, C₁₃, C₁₄, C₁₅, C₁₆, C₁₇, C₁₈, C₁₉ and C₂₀ alkyl; and more preferably, the term "C₁₅-C₂₀ alkyl" adopted in the present application includes C₁₅, C₁₆, C₁₇, C₁₈, C₁₉ and C₂₀ alkyl. Further, the term "C₂-C₅ alkyl" adopted in the present application includes C₂, C₃, C₄ and C₅ alkyl. Further, the term "C₁₆-C₂₀ alkyl" adopted in the present application includes C₁₆, C₁₇, C₁₈, C₁₉ and C₂₀ alkyl.

The term "alkenyl" adopted in the present application is intended to include a hydrocarbon chain having a straight-chain or branched-chain configuration and having one or more carbon-carbon double bonds that can exist at any stable point along the chain. For example, "C₂-C₆ alkenyl" is intended to include C₂, C₃, C₄, C₅ and C₆ alkenyl. Examples of alkenyl includes without limitation ethenyl, propenyl, butenyl, pentenyl and hexenyl.

Specifically, the term "C₆-C₂₀ alkenyl" adopted in the present application includes C₆, C₇, C₈, C₉, C₁₀, C₁₁, C₁₂, C₁₃, C₁₄, C₁₅, C₁₆, C₁₇, C₁₈, C₁₉ and C₂₀ alkenyl; preferably, "C₁₀-C₂₀ alkenyl" adopted in the present application includes C₁₀, C₁₁, C₁₂, C₁₃, C₁₄, C₁₅, C₁₆, C₁₇, C₁₈, C₁₉ and C₂₀ alkenyl; and more preferably, "C₁₅-C₂₀ alkenyl" adopted in the present application includes C₁₅, C₁₆, C₁₇, C₁₈, C₁₉ and C₂₀ alkenyl.

The term "heterocycle" or "heterocyclyl or heterocyclic group" adopted in the present application means a stable monocyclic or bicyclic ring containing heteroatoms or heteroatom groups, which can be saturated, partially unsaturated, or unsaturated (aromatic) and contain carbon atoms and 1, 2, 3 or 4 ring heteroatoms independently selected from N, O and S. Nitrogen atoms can be substituted or unsubstituted. Examples of monocyclic heterocyclyl include without limitation azetidinyl, pyrrolidinyl, oxetanyl, imidazolinyl, oxazolidinyl, isoxazolinyl, thiazolidinyl, isothiazolidinyl, tetrahydrofuranyl, piperidyl, piperazinyl, 2-oxo-piperazinyl, 2-oxo-piperidyl, 2-oxo-pyrrolidinyl, 2-oxo-azepinyl, azepinyl, 1-pyridonyl, 4-piperidonyl, tetrahydropyranyl, morpholinyl, thiomorpholinyl, thiomorpholinyl sulfoxide, thiomorpholinyl sulfone, 1,3-dioxolame and tetrahydro-1,1-dioxo-thienyl. Examples of bicyclic heterocyclyl include without limitation quinuclidinyl. The "heterocycle" adopted by the present invention is preferably a 5-6 membered saturated heterocycle, containing 1 or 2 ring heteroatoms independently selected from N, O and S, more preferably is a piperazine ring.

The term "neutral lipid" adopted in the present application refers to any of the lipids existing in the form of uncharged or neutral zwitterions under physiological pH. Representative neutral lipids include diacylphosphatidylcholine, diacylphosphatidyl ethanolamine, ceramide, sphingomyelin, dihydrosphingomyelin, cephalin and cerebroside. Further, the neutral lipids in the present invention may also be selected from distearoyl phosphatidylcholine (DSPC), dioleoyl phosphatidylcholine (DOPC), dipalmitoyl phosphatidylcholine (DPPC), dioleoyl phosphatidylglycerol (DOPG), dipalmitoyl phosphatidylglycerol (DPPG), dioleoyl phosphatidyl ethanolamine (DOPE), palmitoyl oleoyl phosphatidylcholine (POPC), palmitoyl oleoyl phosphatidyl ethanolamine (POPE) and oleoyl phosphatidyl ethanolamine 4-(N-maleimide methyl)-cyclohexane-1 carboxylate (DOPE-mal), dipalmitoyl phosphatidyl ethanolamine (DPPE), dimyristoyl phosphatidyl ethanolamine (DMPE), distearoyl phosphatidyl ethanolamine (DSPE), 16-O-monomethyl PE, 16-O- dimethyl PE, 18-1-trans PE, 1-stearoyl-2-oleoyl phosphatidyl ethanolamine (SOPE) and 1,2-divaleryl-sn-glycerol-3-phosphoethanolamine (trans-DOPE), preferably, the neutral lipid is selected from DSPC or DOPE.

The term "PEG lipid" adopted in the present application can be selected from PEG-dilauryl glycerol, PEG-dimyristoyl glycerol (PEG-DMG), PEG-dipalmitoyl glycerol, PEG-distearoyl glycerol (PEG-DSPE), PEG-dilauryl glyceramide, PEG-dimyristyl glyceramide, PEG-dipalmitoyl glyceramide and PEG-distearoyl glyceramide, PEG-cholesterol (1-[8'-(cholest-5-ene-3[[β]-oxo)formamido-3',6'-dioxazoctyl]carbamoyl-[ω]-methyl-poly(ethylene glycol), PEG-DMB (3,4-bis-tetradecyloxy phenyl methyl-[ω]-methyl-poly(glycol)ether), mPEG-DMG-2k (dimyristoyl glycerol-polyethylene glycol 2000), 1,2-dimyristoyl-sn-glycerol-3-phosphoethanolamine -N-[methoxy(polyethylene glycol)-2000] (PEG2k-DMG), 1,2-distearoyl-sn-glycerol-3-phosphoethanolamine-N-[methoxy(polyethylene glycol)-2000] (PEG2k-DSPE) (catalog number 880120C, from Avanti Polar Lipids, Alabaster, Alabama, USA), 1,2-distearoyl-sn-glycerol, methoxypolyethylene glycol (PEG2k-DSG; GS-020, NOF Tokyo, Japan), poly(ethylene glycol)-2000-dimethacrylate (PEG2k-DMA) and 1,2-distearoyloxypropyl-3-amine-N-[methoxy(polyethylene glycol)-2000] (PEG2k-DSA), preferably, PEG lipid is selected from mPEG-DMG-2k.

The term "nucleic acid" or "nucleic acid molecule" adopted in the present application will be recognized and understood by those of ordinary skill in the art, for example intended to denote a molecule containing nucleic acid components and preferably is composed of nucleic acid components. The term "nucleic acid molecule" preferably refers to a DNA or RNA molecule and preferably is used synonymously with the term "polynucleotide". Preferably, nucleic acid or nucleic acid molecule is a polymer containing or composed of nucleotide monomers that are covalently connected to each other by the phosphodiester bonds of a sugar/phosphate ester skeleton. Preferably, the nucleic acid molecule is selected from mRNA, siRNA, antisense oligonucleotide (ASO), saRNA or miRNA.

The term "cancer" adopted in the present application refers to a kind of diseases in which a group of cells shows uncontrolled growth (division beyond the normal range), invasion (invading and destroying neighboring tissues) and sometimes metastasis (spreading to other locations in the body through lymph or blood). Most cancers form tumors, i.e., swellings or lesions formed by abnormal growth of cells (called neoplastic cells or tumor cells), but some cancers (such as leukemia) do not. According to the present invention, the term "cancer" includes triple-negative breast cancer, leukemia, seminoma, melanoma, teratoma, lymphoma, neuroblastoma, glioma, rectal cancer, endometrial cancer, kidney cancer, adrenal cancer, thyroid cancer, blood cancer, skin cancer, brain cancer, cervical cancer, bowel cancer, liver cancer, colon cancer, stomach cancer, head and neck cancer, gastrointestinal cancer, lymph node cancer, esophageal cancer, colorectal cancer, pancreatic cancer, ear, nose and throat cancer (ENT), breast cancer, prostate cancer, uterine cancer, ovarian cancer and lung cancer and their metastases. Their examples are triple-negative breast cancer, lung cancer, breast cancer, prostate cancer, colon cancer, renal cell cancer, cervical cancer, or metastasis of the above kinds of cancers or tumors. The term "cancer" according to the present invention also includes cancer metastasis.

The cationic lipid provided by the present invention specifically can be a non-ionized cationic lipid structure, wherein its head group is a protonable heterocyclic ring, the intermediate connecting chain is a non-ionized quaternary ammonium salt, the connecting bond is a biodegradable ester bond, and the tail is a fat chain with a different length, a different degree of saturation and a different number of tail chains. Such structural design enables the non-ionized cationic lipid to achieve higher in vivo mRNA transfection efficiency and only local distribution and expression. Therefore, the cationic lipid provided by the present invention, more specifically, non-ionized cationic lipid compound, has the following advantages: (1) The in vivo and in vitro expression of the LNP preparation made therefrom is superior to that of DOTAP LNP preparation; (2) The LNP preparation made therefrom has a uniform and controllable particle size and lower potential than that of DOTAP preparation at the same ratio, which can alleviate the system safety brought by non-ionized cationic material to a certain extent.

The cationic lipid provided by the present invention specifically may also be an ionizable cationic lipid structure, wherein its head group is a protonable heterocyclic ring, the intermediate connecting chain is a protonable tertiary amine, the connecting bond is a biodegradable ester bond, and the tail is a fat chain with a different length, a different degree of saturation and a different number of tail chains. Such structural design enables the ionizable cationic lipid to achieve higher in vivo mRNA transfection efficiency. Therefore, the cationic lipid provided by the present invention, more specifically, ionizable cationic lipid compound, has the following advantages: (1) According to the characteristics of phospholipid itself and the structure-activity relationship, it is independently designed and synthesized and breaks through foreign patent blockade of materials; (2) Through a certain proportion of the prescription and optimization of the process, the compound LNP preparation in the present invention can produce specific benefits on distribution, which can provide targeted treatment according to different clinical indications and demands; (3) The in vivo transfection effect of the LNP made from the compound is slightly superior or equivalent to that of the LNP made from commercially available head products.

### BRIEF DESCRIPTION OF THE DRAWINGS

FIG. 1: Hela cell transfection results of LNP-1 to LNP-9 in different proportions made from compound 1 and compound 2 provided by the present invention, LNP-19 made from compound 9, LNP-20 made from compound 10 and LNP-21 made from compound 11 relative to LNP-10 made from MC3.
FIG. 2: Animal transfection results of LNP-1 to LNP-3 and LNP-7 to LNP-8 made from compound 1 and compound 2 provided by the present invention, LNP-19 made from compound 9, LNP-20 made from compound 10 and LNP-21 made from compound 11 relative to LNP-10 made from MC3.
FIG. 3: Live animal imaging of LNP-1 to LNP-3 and LNP-7 to LNP-8 made from compound 1 and compound 2 provided by the present invention, LNP-19 made from compound 9, LNP-20 made from compound 10, LNP-21 made from compound 11 and LNP-10 made from MC3.
FIG. 4: Animal transfection results of LNP-11 made from compound 3 provided by the present invention relative to LNP-12 made from DOTAP.
FIG. 5: Live animal imaging of LNP-11 made from compound 3 provided by the present invention and LNP-12 made from DOTAP.
FIG. 6: Animal transfection results of LNP-2 and LNP-22 to LNP-30 made from compound 1 provided by the present invention and LNP-8 and LNP-31 to LNP-34 made from compound 2 relative to LNP-10 made from MC3.
FIG. 7: Live animal imaging of LNP-2 and LNP-22 to LNP-30 made from compound 1 provided by the present invention, LNP-8 and LNP-31 to LNP-34 made from compound 2 and LNP-10 made from MC3.
FIG. 8: Animal transfection results of LNP-8 made from compound 2 provided by the present invention, LNP-35 made from compound 17, LNP-2 made from compound 1 and LNP-36 made from compound 18 relative to LNP-10 made from MC3.
FIG. 9: Live animal imaging of LNP-8 made from compound 2 provided by the present invention, LNP-35 made from compound 17, LNP-2 made from compound 1, LNP-36 made from compound 18, and LNP-10 made from MC3.
FIG. 10: Results of immunogenicity of LNP-37 made from compound 1 provided by the present invention and LNP-38 made from commercially available SM-102 in mice.

### DETAILED DESCRIPTION

Below the technical solution of the present invention will be further described in detail by referring to specific embodiments. It should be understood that the following embodiments are intended to exemplarily describe and illustrate the present invention only and should not be understood to limit the scope of protection of the present invention. Any technical solution implemented based on the content of the present invention shall fall within the scope of protection intended by the present invention.

Unless otherwise stated, the raw materials and reagents used in the following embodiments are commercially available or can be prepared by known methods.

### Examples

### Synthesis of different cationic lipids

### Example 1: Preparation of compound 1

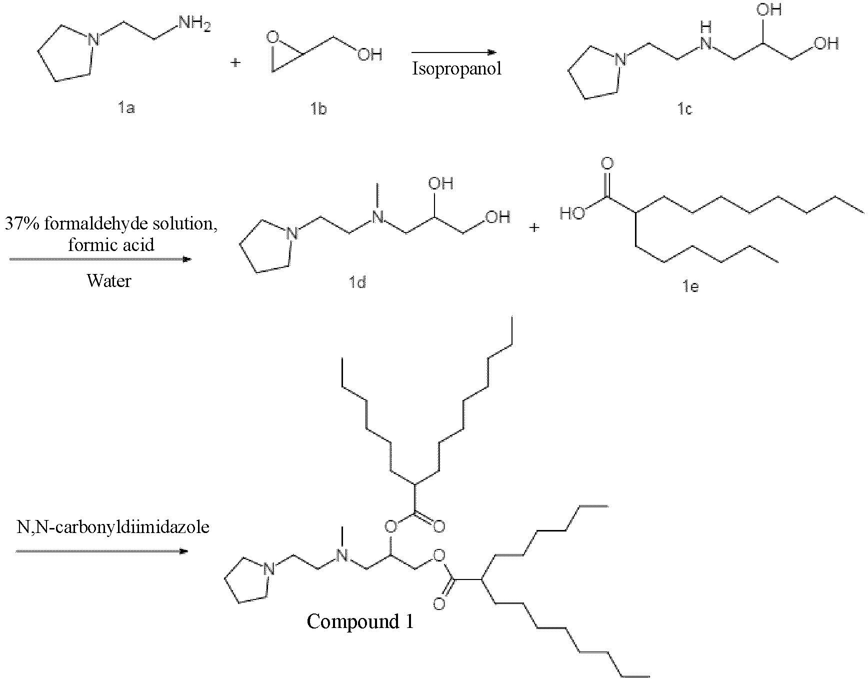

**Synthesis of compound 1c**: Compound 1a (18.5g) was dissolved in 180ml of isopropanol, stirred at 50-60°C, compound 1b (4g of glycidol) was added dropwise, reacted under temperature control, and after TLC indicated the reaction completion, the reaction system was concentrated at 50-60°C under reduced pressure to obtain 10.21g of brown oil.

**Synthesis of compound 1d**: Compound 1c (10.21g) was added into the reaction bottle, 100g of water was added for dissolution, and 1.8g of formic acid and 3.2g of 37% formaldehyde solution were subsequently added dropwise. After the completion of addition, the inner temperature was raised to 40-50°C, and after TLC indicated the reaction completion, pH was regulated to 8-9 by using sodium hydroxide, the reaction was extracted with **dichloromethane, and the organic phase was concentrated under reduced pressure to obtain 9.0g of yellow oil.**

**Synthesis of compound 1**: 14.58g of compound 1e was stirred and dissolved with 140ml of trichloromethane, N,N-carbonyldiimidazole and 5g of compound 1d were subsequently added, stirred and reacted at 50-60°C, and after TLC indicated the reaction completion, the solvent was removed by means of rotary evaporation and reduced pressure distillation. The reaction was purified through separation on columns (silica gel column, eluent DCM:MeOH=100:1 (volume ratio)) to obtain 3.4g of **compound 1,** which is pale-yellow oil. 1H NMR (400 MHz, CDCl₃) δ 5.18-5.16 (m, 1H), 4.42-4.40 (m, 1H), 4.07-4.04 (m, 1H), 2.65-2.54 (m, 7H), 2.53 (t, J = 2.7 Hz, 2H), 2.34-2.29 (m, 5H), 1.80 (s, 4H), 1.56-1.44 (m, 8H), 1.26 (s, 41H), 0.88 (t, J = 7.0 Hz, 12H). MS m/z (ESI): 680.8 [M+H]⁺.

### Example 2: Preparation of compound 2

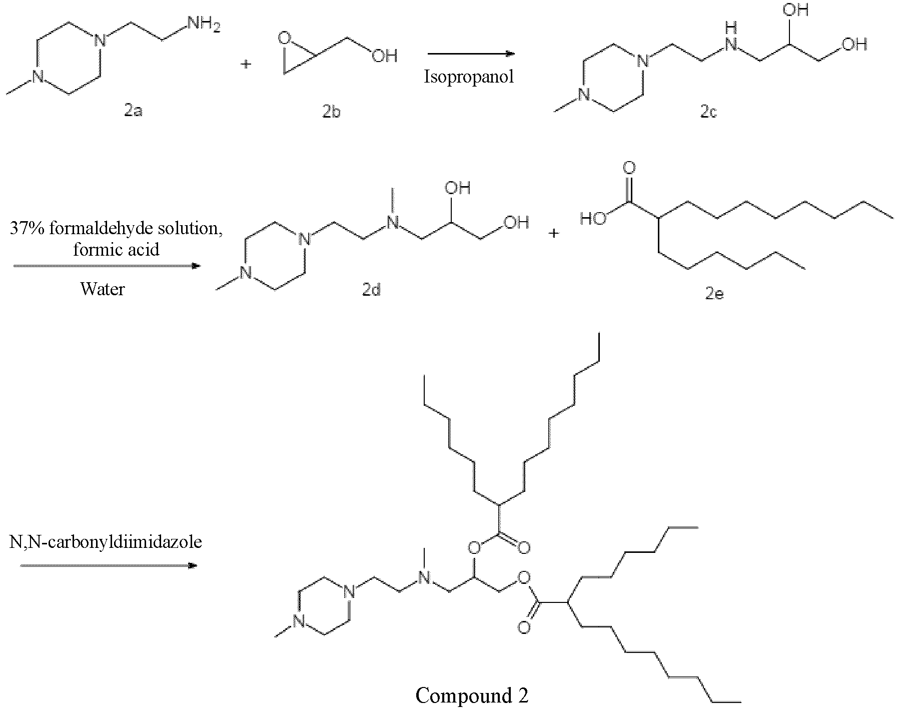

**Synthesis of compound 2c:** Compound 2a (22g) was dissolved in 220ml of isopropanol, compound 2b (4g of glycidol) was added dropwise at 50-60°C, reacted under temperature control, and after TLC indicated the reaction completion, the reaction system was concentrated at 60-70°C under reduced pressure to obtain 11.8g of pale-yellow oil.

**Synthesis of compound 2d:** Compound 2c (11.8g) was added into the reaction bottle, 110g of water was added for dissolution, and 1.8g of formic acid and 3.2g of 37% formaldehyde solution were subsequently added dropwise. After the completion of addition, the inner temperature was raised to 40-50°C, and after TLC indicated the reaction completion, the pH was regulated to 8-9 by using sodium hydroxide, the reaction was extracted with dichloromethane, and the organic phase was concentrated under reduced pressure to obtain 4.0g of yellow oil.

**Synthesis of compound 2:** 10.2g of compound 2e was stirred and dissolved with 100ml of trichloromethane, N,N-carbonyldiimidazole and 4g of compound 2d were subsequently added, stirred and reacted at 50-60°C, and after TLC indicated the reaction completion, the solvent was removed by means of rotary evaporation and reduced pressure distillation. The reaction was purified through separation on columns (silica gel column, eluent DCM:MeOH=100:1 (volume ratio)) to obtain 2.29g of **compound 2,** which is pale-yellow oil. 1H NMR (400 MHz, CDCl₃) δ 5.18-5.14 (quint, J = 7.1 Hz,1H), 4.42-4.39 (d,1H), 4.07-4.04 (d, J = 7.1 Hz,1H), 2.58-2.43 (m,8H), 2.28(s,6H), 1.60-1.56 (m,4H), 1.43-1.42 (m,4H), 1.26 (s, 42H), 0.88 (t, J = 7.0 Hz, 12H). MS m/z (ESI): 709.5 [M+H]⁺.

### Example 3: Preparation of compound 3

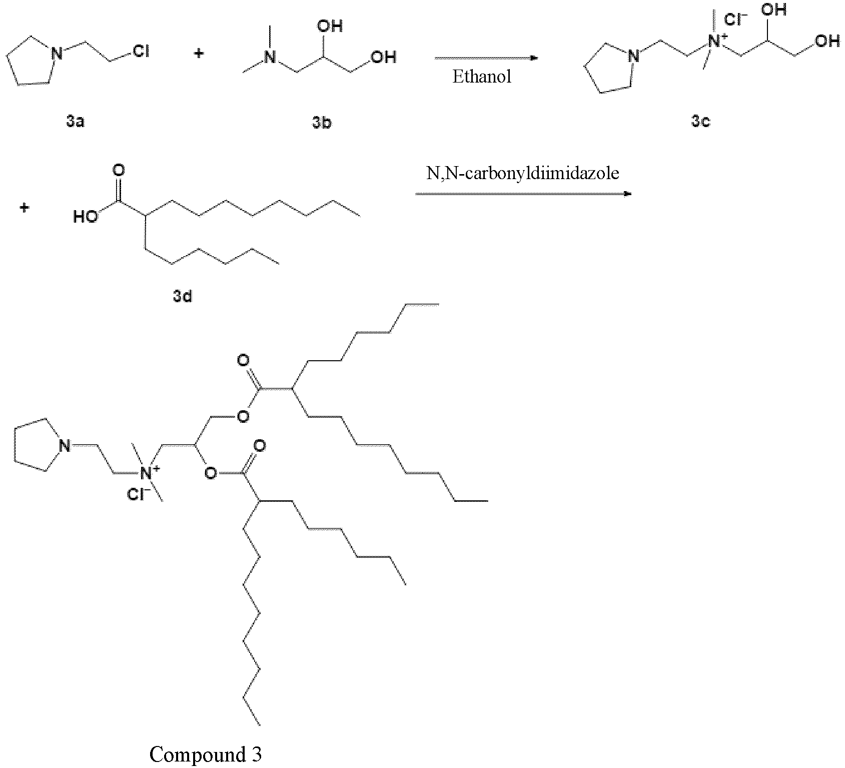

**Synthesis of compound** 3c: 3g of compound 3b was weighed, and dissolved in 30ml of ethanol, compound 3a was added, the temperature was raised to 45-50°C, and after TLC indicated the reaction completion, the reaction was concentrated under reduced pressure to obtain 4.81g of pale-yellow oil.

**Synthesis of compound** 3: 11.6g of compound 3d was stirred and dissolved with 110ml of dichloromethane, N,N-carbonyldiimidazole and 4.81g of compound 3c were subsequently added, and stirred and reacted at 50-60°C. After TLC indicated the reaction completion, the solvent was removed by means of rotary evaporation and reduced pressure distillation. The reaction was purified through separation on columns (silica gel column, eluent DCM:MeOH=50:1; DCM:MeOH=30:1 (volume ratio)) to obtain 1.13g of **compound 3,** which is pale-yellow oil. 1H NMR (400 MHz, CDCl₃) δ 5.62 (s, 1H), 4.67-4.50 (m, 2H), 4.10 (dd, *J* = 12.3, 5.8 Hz, 1H), 3.95 (s, 2H), 3.77 (dd, *J* = 14.3, 7.8 Hz, 1H), 3.52 (d, *J* = 5.0 Hz, 6H), 3.20-2.83 (m, 2H), 2.54 (t, *J* = 48.6 Hz, 4H), 2.41-2.24 (m, 3H), 1.79 (s, 4H), 1.68-1.34 (m, 8H), 1.26 (s, 41H), 0.88 (t, *J =* 6.6 Hz, 12H). MS m/z (ESI): 693.6[M-Cl]⁺.

### Example 4: Preparation of compound 4

**Compound 4** is prepared by the same method as for compound 3. ¹H NMR (400 MHz, CDCl₃) δ 5.15 (1H, tt, J = 6.0, 5.7 Hz), 4.47-4.36 (2H, 4.42 (d, J = 6.0 Hz), 3.37-3.24 (4H, 3.30 (d, J = 5.7 Hz), 3.14-2.83 (12H, 2.89 (t, J = 6.8 Hz), 2.33-2.20(4H, 2.26 (t, J = 7.4 Hz), 1.99-1.79 (4H), 1.63-1.49 (4H), 1.34-1.17 (24H, 1.23 (quint, J = 7.0 Hz), 0.90 (t, J = 6.8 Hz,6H). MS m/z (ESI): 525.83 [M-Cl]+.

### Example 5: Preparation of compound 5

**Compound 5** is prepared by the same method as for compound 4. ¹H NMR (400 MHz, CDCl₃) δ 5.42-5.31 (m, 5H), 5.25-5.15 (m, 1H), 4.36 (dd, J = 11.9, 3.1 Hz, 1H), 4.03 (ddd, J = 19.0, 14.3, 8.6 Hz, 4H), 3.96-3.79 (m, 4H), 3.32 (d, J = 5.9 Hz, 4H), 3.03-2.86 (m, 2H), 2.61 (qd, J = 13.4, 6.4 Hz, 3H), 2.44 (d, J = 10.7 Hz, 3H), 2.40-2.35 (m, 2H), 2.34-2.16 (m, 7H), 2.03 (d, J = 5.6 Hz, 10H), 1.64-1.59 (m, 4H), 1.30 (d, J = 13.5 Hz, 41H), 0.89 (d, J = 7.0 Hz, 6H).MS m/z (ESI): 745.67 [M-Cl]+.

### Example 6: Preparation of compound 6

**Compound 6** is prepared by the same method as for compound 3. ¹H NMR (400 MHz, CDCl₃) δ 5.64 (s, 1H), 5.45-5.28 (m, 8H), 4.61-4.44 (m, 2H), 4.13 (dd, *J* = 12.1, 5.8 Hz, 1H), 3.92 (t, *J* = 11.4 Hz, 3H), 3.51 (t, *J* = 8.2 Hz, 6H), 3.14-2.96 (m, 2H), 2.84-2.71 (m, 4H), 2.63 (s, 4H), 2.42-2.28 (m, 4H), 2.06 (q, *J* = 6.7 Hz, 8H), 1.90-1.74 (m, 4H), 1.70-1.54 (m, 4H), 1.43-1.23 (m, 28H), 0.90 (t, *J =* 6.8 Hz, 6H).MS m/z (ESI): 741.1 [M-Cl]+.

### Example 7: Preparation of compound 7

**Compound 7** is prepared by the same method as for compounds 3 and 4. 1-Boc-4-(2-chloroethyl)piperazine was used as starting material and lastly, Boc is removed to obtain compound 7. ¹H NMR (400 MHz, CDCl₃) δ 5.65 (s, 1H), 5.42-5.31 (m, 4H), 4.58-4.46 (m, 2H), 4.13 (dd, *J* = 12.1, 5.9 Hz, 1H), 3.97 (s, 1H), 3.88 (dd, *J* = 14.4, 8.6 Hz, 2H), 3.48 (d, *J* = 2.9 Hz, 6H), 2.97 (s, 3H), 2.91 (d, *J* = 5.2 Hz, 2H), 2.64 (s, 4H), 2.36 (dt, *J =* 15.4, 7.8 Hz, 4H), 2.05 (t, *J* = 15.9 Hz, 15H), 1.63 (d, *J* = 7.0 Hz, 4H), 1.37-1.23 (m, 48H), 0.90 (t, *J* = 6.7 Hz, 6H).MS m/z (ESI): 761.37 [M-Cl]+.

### Example 8: Preparation of compound 8

**Compound 8** is prepared by the same method as for compound 7. ¹H NMR (400 MHz, CDCl₃) δ 5.65 (s, 1H), 5.45-5.29 (m, 8H), 4.53-4.37 (m, 2H), 4.11 (dt, *J =* 16.3, 8.2 Hz, 1H), 3.88 (ddd, *J*= 31.9, 14.2, 8.7 Hz, 3H), 3.45 (s, 6H), 2.88 (dd, *J* = 12.4, 8.0 Hz, 6H), 2.79 (t, *J* = 6.3 Hz, 4H), 2.57 (d, *J* = 31.5 Hz, 4H), 2.43-2.26 (m, 4H), 2.07 (dd, *J =* 13.6, 6.7 Hz, 10H), 1.62 (d, *J =* 7.0 Hz, 4H), 1.41-1.10 (m, 33H), 0.91 (t, *J =* 6.8 Hz, 6H). MS m/z (ESI): 756.97 [M-Cl]+.

### Example 9: Preparation of compound 9

**Compound** 9 is prepared by the same method as for compound 1. ¹H NMR (400 MHz, CDCl₃) δ 7.30-7.09 (m, 1H), 5.44-5.26 (m, 11H), 5.30-5.12 (m, 1H), 4.35 (dd, *J* = 11.9, 3.1 Hz, 1H), 4.09 (dd, *J* = 11.9, 6.4 Hz, 1H), 2.85-2.69 (m, 12H), 2.69-2.62 (m, 2H), 2.55 (d, *J =* 6.5 Hz, 2H), 2.35-2.27 (m, 8H), 2.23 (t, *J* = 7.6 Hz, 1H), 2.06 (q, *J* = 6.7 Hz, 11H), 1.86 (s, 4H), 1.67-1.56 (m, 5H), 1.39-1.27 (m, 38H), 0.90 (t, *J =* 6.8 Hz, 6H). MS m/z (ESI): 728.10 [M+H]⁺.

### Example 10: Preparation of compound 10

**Compound 10** is prepared by the same method as for compound 2. ¹H NMR (400 MHz, CDCl₃) δ 5.46-5.28 (m, 9H), 5.19 (qd, *J* = 6.4, 3.1 Hz, 1H), 4.37 (dd, *J* = 11.9, 3.0 Hz, 1H), 4.11 (dd, *J* = 11.9, 6.5 Hz, 1H), 2.93 (d, *J* = 6.0 Hz, 1H), 2.85-2.63 (m, 5H), 2.68-2.35 (m, 15H), 2.40-2.24 (m, 12H), 2.06 (q, *J* = 6.7 Hz, 10H), 1.71-1.52 (m, 5H), 1.42-1.22 (m, 37H), 0.90 (t, *J* = 6.8 Hz, 6H). MS m/z (ESI): 757.24[M+H]⁺.

### Example 11: Preparation of compound 11

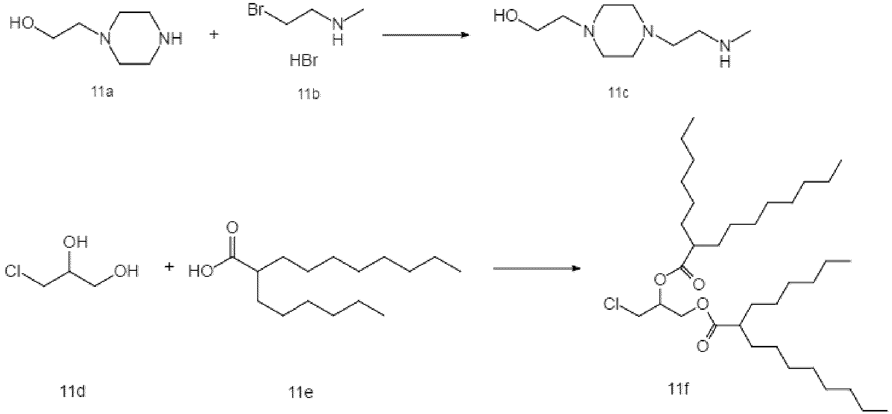

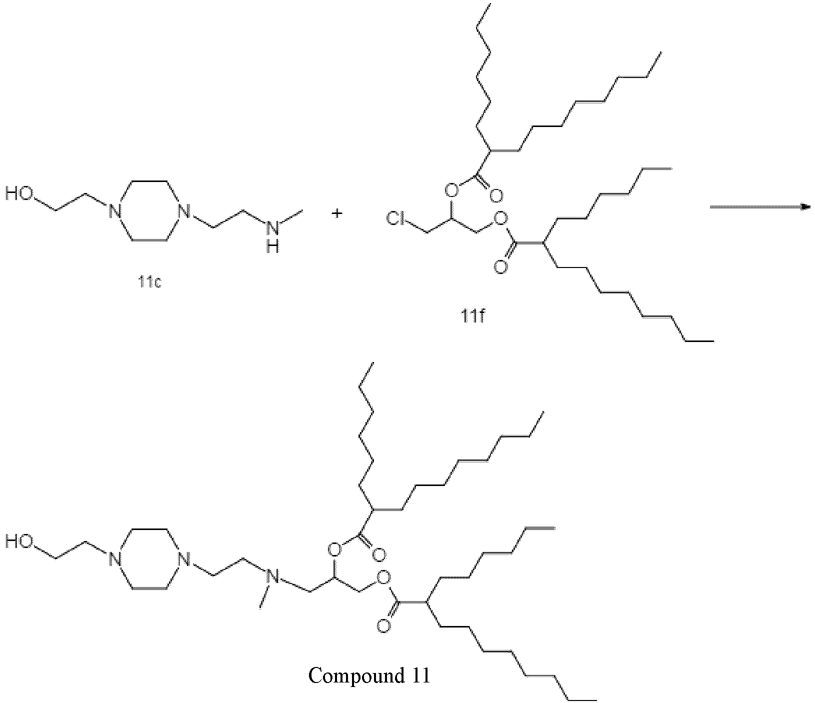

**Synthesis of compound 11c:** Compound 11a (2.7g) was dissolved in 100ml of acetonitrile, 11.5g of potassium carbonate and 0.345g of potassium iodide were added, stirring was started, and the temperature was raised to 50°C. 11b (5g) was added into the foregoing 11a system, reacted under heat preservation, and after TLC indicated the reaction completion, the inorganic salt was removed by filtration and the mother liquor was concentrated under reduced pressure to obtain pale-yellow oil, 4.23g.

**Synthesis of compound 11f**: 11e (30g) was dissolved in 300ml of dichloromethane, oxalyl chloride (29.7g) was added dropwise to the dichloromethane system of the foregoing 11e at room temperature, during which a small amount of air bubbles and smoke were produced, stirred and reacted at room temperature for 1h after the completion of addition, and concentrated under reduced pressure after the end of reaction to obtain 31.68g of pale-yellow oil. 11d (6g) was dissolved in 60ml of dichloromethane, 16.4g of triethylamine was added, stirred at room temperature, 31.68g of the light-yellow oil obtained above was added dropwise into the foregoing system, stirred and reacted for 2h after the addition, the reaction liquid was washed with an equal volume of water for three times after the end of reaction, the organic phase was collected, dried and concentrated to obtain 11f (34.9g) of crude oil, and the crude product was purified through column chromatography (dichloromethane) to obtain 22.44g of 11f oil.

**Synthesis of compound 11:** 3.5g of compound 11c was stirred and dissolved with 150ml of N,N-dimethylformamide and 12.6g of potassium carbonate and 0.42g of potassium iodide were added. The temperature was raised to 40°C. 11f (10.5g) was added dropwise into the 11c system, and reacted at 40-50°C under temperature control after the addition, and after TLC indicated the reaction completion, the insoluble substances were filtered away, the mother liquor was concentrated under reduced pressure to obtain 13.54g of pale-yellow oil crude product, and purified through column chromatography to obtain 400mg of **compound 11,** which is pale-yellow oil. ¹H NMR (400 MHz, CDCl₃) δ 5.08-4.67 (m, 1H), 3.71-3.60 (m, 1H), 3.50 (dt, *J* = 33.6, 7.2 Hz, 1H), 3.03 (d, *J* = 15.8 Hz, 1H), 2.69-2.47 (m, 6H), 1.61 (dd, *J* = 13.3, 7.7 Hz, 1H), 1.43 (dd, *J* = 13.2, 6.1 Hz, 2H), 1.25 (s, 14H), 0.87 (t, *J* = 6.2 Hz, 4H). MS m/z (ESI): 739.04 [M+H]⁺.

### Example 12: Preparation of compound 17

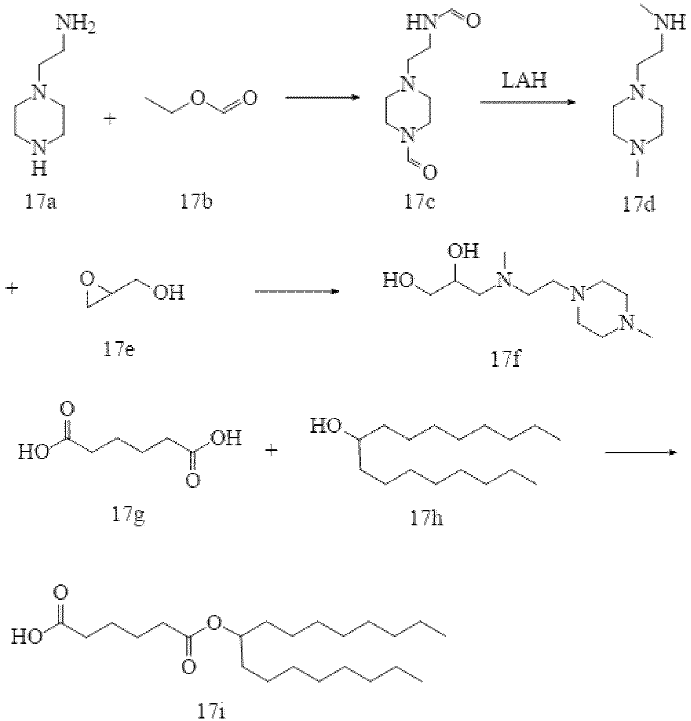

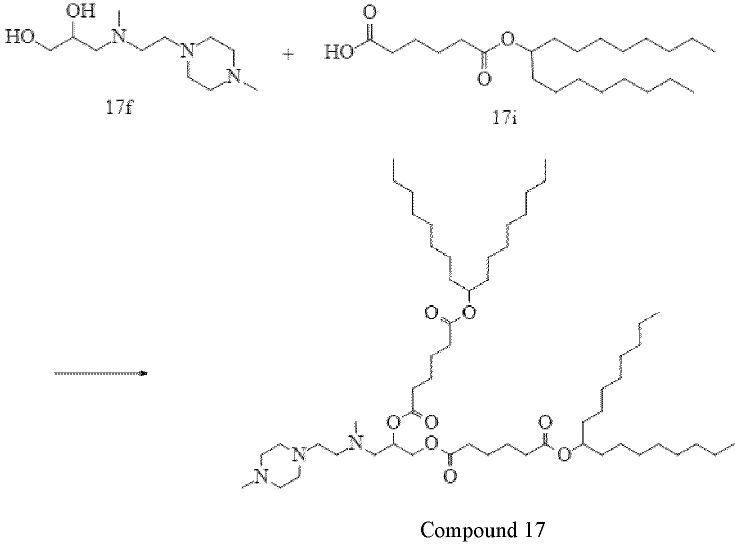

**Synthesis of compound 17c:** Compound 17a (5.0g) was added into 17b (50ml), reacted under reflux for 8h and after TLC indicated the reaction completion, concentrated under reduced pressure to obtain pale-yellow oil (6.49g), which was directly used in the next step of the reaction.

**Synthesis of compound 17d:** Compound 17c (6.49g) was dissolved in 70ml of tetrahydrofuran, lithium aluminum hydride (LAH) (1.5eq) was added in batches at room temperature, air and heat release was observed, the temperature was controlled at 30-40°C using an ice-water bath, and after the completion of addition, the temperature was raised to react under reflux, and after TLC indicated the reaction completion, the reaction liquid was cooled to 5-10°C, water, sodium hydroxide and water were added subsequently to quench the reaction, filtered directly after quenching, the filter cake was washed with tetrahydrofuran, the filtrate was concentrated under reduced pressure to obtain 5.5g of yellow oil, which was directly used in the next step of the reaction.

**Synthesis of compound 17f:** Compound 17d (5.50g) was dissolved in 60ml of isopropanol, 17e (2eq) of isopropanol solution was added dropwise into 17d reaction liquid at room temperature, reacted at room temperature overnight after the completion of addition, and after TLC indicated the reaction completion, purified through separation on columns (silica gel column, eluent dichloromethane: methanol=40:1 (volume ratio)) to obtain 3.5g of orange red oil.

**Synthesis of compound 17i:** Compound 17g (20g) was dissolved in dichloromethane, 17h (0.5eq), N,N-diisopropylethylamine (DIPEA) (1eq), 4-dimethylaminopyridine (DMAP) (0.5eq) and 1-ethyl-(3-dimethylaminopropyl) carbamide diimide hydrochloride (EDCI) (1eq) were added subsequently at room temperature, reacted overnight under stirring at room temperature after the completion of addition, and after TLC indicated the reaction completion, diluted hydrochloric acid was directly added to the reaction liquid for washing and layering, the organic phase was washed with an equal volume of purified water and sodium chloride aqueous solution, respectively, each once, the organic phase was concentrated under reduced pressure to obtain oil, and purified through separation on columns (silica gel column, eluent DCM:MeOH=100:1 (volume ratio)) to obtain 21.2g of pale-yellow oil.

**Synthesis of compound 17:** Compound 17i (4g) was dissolved in 20ml of dichloromethane, a few drops of DMF was added, then cooled in an ice-water bath to 0-5°C, oxalyl chloride (1.5eq) was added dropwise, reacted at room temperature after the completion of addition, and after TLC indicated the reaction completion, directly concentrated under reduced pressure till dryness. 17f (0.5eq) was dissolved in dichloromethane, triethylamine (2eq) was added, cooled in an ice bath till 0-5°C, the foregoing substance was dried through concentration under reduced pressure in an appropriate volume of dichloromethane, then the obtained solution was added dropwise to the reaction system of 17f, reacted overnight at room temperature after the completion of addition, and after TLC indicated the reaction completion, the reaction liquid was washed with a citric acid aqueous solution and saturated sodium chloride aqueous solution, respectively each once, the organic phase was concentrated to obtain pale-yellow oil, and purified through separation on columns (silica gel column, eluent DCM:MeOH=80:1 (volume ratio)) to obtain 1.21g of pale-yellow oil. ¹H NMR (400 MHz, CDCl₃) δ 5.51 (d, J = 4.9 Hz, 1H), 4.86 (dd, J = 6.1, 1.4 Hz, 2H), 4.40 (s, 1H), 4.07 (dd, J = 12.1, 5.4 Hz, 1H), 3.96-3.28 (m, 6H), 3.16 (s, 4H), 2.82 (dd, J = 13.0, 7.4 Hz, 10H), 2.50-2.25 (m, 8H), 1.66 (s, 8H), 1.51 (d, J = 5.7 Hz, 8H), 1.28 (d, J = 15.4 Hz, 48H), 0.89 (t, J = 6.8 Hz, 12H). MS m/z (ESI): 964.82 [M+H]⁺.

### Example 13: Preparation of compound 18

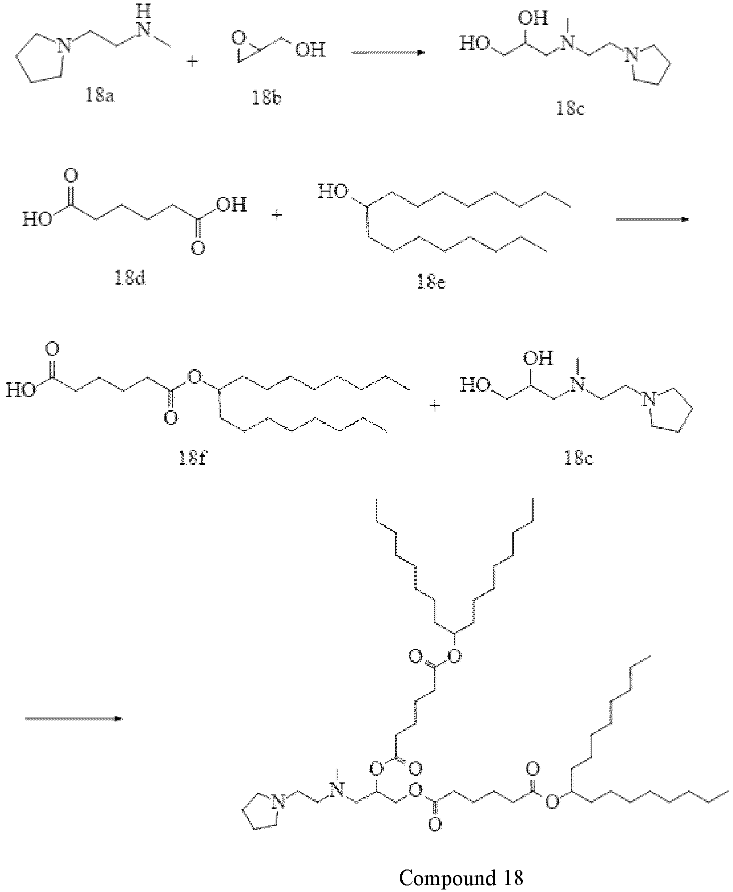

**Synthesis of compound** 18c: Compound 18a (6.4g) was dissolved in 100ml of ethanol, compound 18b (3.7g of glycidol) was added dropwise under reflux, reacted under temperature control, and after TLC indicated the reaction completion, concentrated under reduced pressure to obtain pale-yellow oil, and purified through separation on columns (silica gel column, eluent DCM:MeOH=100:1 (volume ratio)) to obtain 4.5g of oil.

**Synthesis of compound 18f:** Compound 18d (20g) was dissolved in dichloromethane, 18e (0.5eq), DIPEA (1eq), DMAP (0.5eq) and EDCI (1eq) were added subsequently at room temperature, reacted overnight under stirring at room temperature after the completion of addition, and after TLC indicated the reaction completion, diluted hydrochloric acid was directly added to the reaction liquid for washing and layering, the organic phase was washed with an equal volume of purified water and sodium chloride aqueous solution in turn each once, the organic phase was concentrated under reduced pressure to obtain oil and purified through separation on columns (silica gel column, eluent DCM:MeOH=100:1 (volume ratio)) to obtain 24.2g of pale-yellow oil.

**Synthesis of compound 18:** Compound 18f (0.8g) was dissolved in 10ml of dichloromethane, 18c (0.4g), DIEA(0.39g), DMAP(0.1g) and EDCI(0.5g) were added subsequently at room temperature, reacted overnight under stirring at room temperature after the completion of addition, and after TLC indicated that only a small amount of 18f was not fully reacted, the reaction liquid was concentrated under reduced pressure to obtain oil, and purified through separation on columns (silica gel column, eluent petroleum ether: ethyl acetate =1:10 (volume ratio)) to obtain 0.6g of pale-yellow oil. ¹H NMR (400 MHz, CDCl₃) δ 5.26-5.14 (m, 1H), 4.85 (d, *J* = 1.4 Hz, 2H), 4.35 (dd, *J* = 12.0, 3.1 Hz, 1H), 4.13-4.03 (m, 1H), 2.59 (s, 10H), 2.33 (dd, *J =* 11.9, 4.9 Hz, 11H), 2.13 (s, 4H), 1.66 (s, 8H), 1.50 (d, *J* = 5.5 Hz, 8H), 1.32-1.22 (m, 48H), 0.88 (t, *J* = 6.8 Hz, 12H). MS m/z (ESI): 935.96 [M+H]⁺.

### Preparation of different lipid nanoparticles (LNP) from the cationic lipids prepared above

### Preparation Example 1: Preparation of lipid nanoparticles containing 25 mol% compound 1 (LNP-1)

A certain amount of compound 1, DSPC (Lipoid), cholesterol (Nanjing Luye) and mPEG-DMG-2k (Sinopeg) were weighed at a molar ratio of 25: 43.5: 30: 1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-1 preparation.

### Preparation Example 2: Preparation of lipid nanoparticles containing 50 mol% compound 1 (LNP-2)

A certain amount of compound 1, DSPC, cholesterol and mPEG-DMG-2k were weighed at a molar ratio of 50:10:38.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-2 preparation.

### Preparation Example 3: Preparation of lipid nanoparticles containing 25 mol% compound 2 (LNP-3)

A certain amount of compound 2, DSPC, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-3 preparation.

### Preparation Example 4: Preparation of lipid nanoparticles containing 31.5 mol% compound 2 (LNP-4)

A certain amount of compound 2, DSPC, cholesterol and mPEG-DMG-2k were weighed at a molar ratio of 31.5:10:56:2.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-4 preparation.

### Preparation Example 5: Preparation of lipid nanoparticles containing 43.3 mol% compound 2 (LNP-5)

A certain amount of compound 2, DSPC, cholesterol and mPEG-DMG-2k were weighed at a molar ratio of 43.3:8.7:46.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-5 preparation.

### Preparation Example 6: Preparation of lipid nanoparticles containing 46.3 mol% compound 2 (LNP-6)

A certain amount of compound 2, DSPC, cholesterol and mPEG-DMG-2k were weighed at a molar ratio of 46.3:9.5:42.7:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-6 preparation.

### Preparation Example 7: Preparation of lipid nanoparticles containing 50 mol% compound 2 (LNP-7)

A certain amount of compound 2, DSPC, cholesterol and mPEG-DMG-2k were weighed at a molar ratio of 50:18.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-7 preparation.

### Preparation Example 8: Preparation of lipid nanoparticles containing 50 mol% compound 2 (LNP-8)

A certain amount of compound 2, DSPC, cholesterol and mPEG-DMG-2k were weighed at a molar ratio of 50:10:38.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-8 preparation.

### Preparation Example 9: Preparation of lipid nanoparticles containing 57.1 mol% compound 2 (LNP-9)

A certain amount of compound 2, DSPC, cholesterol and mPEG-DMG-2k were weighed at a molar ratio of 57.1:7.1:34.3:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-9 preparation.

### Preparation comparison example 10: Preparation of lipid nanoparticles containing 50 mol% MC3 (LNP-10)

A certain amount of (dilinoleyl)methyl 4-(N,N-dimethylamino)butanoate (DLin-MC3-DMA/MC3, SHOCHEM), DSPC, cholesterol and mPEG-DMG-2k were weighed at a molar ratio of 50:10:38.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-10 preparation.

### Preparation Example 11: Preparation of lipid nanoparticles containing 25 mol% compound 3 (LNP-11)

A certain amount of compound 3, DSPC, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-11 preparation.

### Preparation comparison example 12: Preparation of lipid nanoparticles containing 25 mol% DOTAP (LNP-12)

A certain amount of (2,3-dioleoyl-propyl)-trimethyl ammonium chloride (DOTAP, Lipoid), DSPC, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-12 preparation.

### Preparation Example 13: Preparation of lipid nanoparticles containing 25 mol% compound 4 (LNP-13)

A certain amount of compound 4, DSPC, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-13 preparation.

### Preparation Example 14: Preparation of lipid nanoparticles containing 25 mol% compound 5 (LNP-14)

A certain amount of compound 5, DSPC, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-14 preparation.

### Preparation Example 15: Preparation of lipid nanoparticles containing 25 mol% compound 6 (LNP-15)

A certain amount of compound 6, DSPC, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-15 preparation.

### Preparation Example 16: Preparation of lipid nanoparticles containing 25 mol% compound 6 (LNP-16)

A certain amount of compound 6, DOPE, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-16 preparation.

### Preparation Example 17: Preparation of lipid nanoparticles containing 25 mol% compound 7 (LNP-17)

A certain amount of compound 7, DSPC, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-17 preparation.

### Preparation Example 18: Preparation of lipid nanoparticles containing 25 mol% compound 8 (LNP-18)

A certain amount of compound 8, DSPC, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-18 preparation.

### Preparation Example 19: Preparation of lipid nanoparticles containing 25 mol% compound 9 (LNP-19)

A certain amount of compound 9, DSPC, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-19 preparation.

### Preparation Example 20: Preparation of lipid nanoparticles containing 25 mol% compound 10 (LNP-20)

A certain amount of compound 10, DSPC, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-20 preparation.

### Preparation Example 21: Preparation of lipid nanoparticles containing 25 mol% compound 11 (LNP-21)

A certain amount of compound 11, DOPE, cholesterol and mPEG-DMG-2k were weighed at a molar ratio of 25:43.5:30:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-21 preparation.

### Preparation Example 22: Preparation of lipid nanoparticles containing 45 mol% compound 1 (LNP-22)

A certain amount of compound 1, DSPC, cholesterol and mPEG-DMG-2k were weighed at a molar ratio of 45:10:43.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-22 preparation.

### Preparation Example 23: Preparation of lipid nanoparticles containing 45 mol% compound 1 (LNP-23)

A certain amount of compound 1, DSPC, cholesterol and mPEG-DMG-2k were weighed at a molar ratio of 45:15:38.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-23 preparation.

### Preparation Example 24: Preparation of lipid nanoparticles containing 45 mol% compound 1 (LNP-24)

A certain amount of compound 1, DSPC, cholesterol and mPEG-DMG-2k were weighed at a molar ratio of 45:18.5:35:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-24 preparation.

### Preparation Example 25: Preparation of lipid nanoparticles containing 45 mol% compound 1 (LNP-25)

A certain amount of compound 1, DSPC, cholesterol and mPEG-DMG-2k were weighed at a molar ratio of 45:22.5:31:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-25 preparation.

### Preparation Example 26: Preparation of lipid nanoparticles containing 43.3 mol% compound 1 (LNP-26)

A certain amount of compound 1, DSPC, cholesterol and mPEG-DMG-2k were weighed at a molar ratio of 43.3:8.7:46.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-26 preparation.

### Preparation Example 27: Preparation of lipid nanoparticles containing 40 mol% compound 1 (LNP-27)

A certain amount of compound 1, DSPC, cholesterol and mPEG-DMG-2k were weighed at a molar ratio of 40:12.5:46:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-27 preparation.

### Preparation Example 28: Preparation of lipid nanoparticles containing 40 mol% compound 1 (LNP-28)

A certain amount of compound 1, DSPC, cholesterol and mPEG-DMG-2k were weighed at a molar ratio of 40:15:43.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-28 preparation.

### Preparation Example 29: Preparation of lipid nanoparticles containing 40 mol% compound 1 (LNP-29)

A certain amount of compound 1, DSPC, cholesterol and mPEG-DMG-2k were weighed at a molar ratio of 40:20:38.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-29 preparation.

### Preparation Example 30: Preparation of lipid nanoparticles containing 36.5 mol% compound 1 (LNP-30)

A certain amount of compound 1, DSPC, cholesterol and mPEG-DMG-2k were weighed at a molar ratio of 36.5:16:46:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-30 preparation.

### Preparation Example 31: Preparation of lipid nanoparticles containing 45 mol% compound 2 (LNP-31)

A certain amount of compound 2, DSPC, cholesterol and mPEG-DMG-2k were weighed at a molar ratio of 45:10:43.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-31 preparation.

### Preparation Example 32: Preparation of lipid nanoparticles containing 45 mol% compound 2 (LNP-32)

A certain amount of compound 2, DSPC, cholesterol and mPEG-DMG-2k were weighed at a molar ratio of 45:15:38.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-32 preparation.

### Preparation Example 33: Preparation of lipid nanoparticles containing 45 mol% compound 2 (LNP-33)

A certain amount of compound 2, DSPC, cholesterol and mPEG-DMG-2k were weighed at a molar ratio of 45:18.5:35:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-33 preparation.

### Preparation Example 34: Preparation of lipid nanoparticles containing 40 mol% compound 2 (LNP-34)

A certain amount of compound 2, DSPC, cholesterol and mPEG-DMG-2k were weighed at a molar ratio of 40:20:38.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-34 preparation.

### Preparation Example 35: Preparation of lipid nanoparticles containing 50 mol% compound 17 (LNP-35)

A certain amount of compound 17, DSPC, cholesterol and mPEG-DMG-2k were weighed at a molar ratio of 50:10:38.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-35 preparation.

### Preparation Example 36: Preparation of lipid nanoparticles containing 50 mol% compound 18 (LNP-36)

A certain amount of compound 18, DSPC, cholesterol and mPEG-DMG-2k were weighed at a molar ratio of 50:10:38.5:1.5 and a lipid ethanol solution with a total lipid concentration of 23mg/mL was prepared. FFluc mRNA (SEQ ID NO.1) was diluted with pH4.0 malic acid buffer and the mass ratio of lipids to FFluc mRNA was 20:1. The foregoing lipid ethanol solution and the FFluc mRNA aqueous solution were mixed at a volume ratio of 1:3 on a microfluidic device (Micro & Nano, INano L) at 50°C and a flow rate of 10mL/min, and the obtained LNP was dialyzed with pH7.4 PBS for 24h, to obtain the ultimate LNP-36 preparation.

### Preparation Example 37: Preparation of lipid nanoparticles containing 36.5 mol% compound 1 (LNP-37)

A certain amount of compound 1, DSPC, cholesterol and mPEG-DMG-2k were weighed at a molar ratio of 36.5:16:46:1.5 and a lipid ethanol solution with a total lipid concentration of 23mg/mL was prepared. HPV mRNA (SEQ ID NO.2) was diluted with pH4.0 malic acid buffer and the mass ratio of lipids to HPV mRNA was 20:1. The foregoing lipid ethanol solution and the HPV mRNA aqueous solution were mixed at a volume ratio of 1:3 on a microfluidic device (Micro & Nano, INano L) at 50°C and a flow rate of 12mL/min, and the obtained LNP was dialyzed with pH7.4 PBS for 24h, to obtain the ultimate LNP-37 preparation.

### Preparation comparison example 38: Preparation of lipid nanoparticles containing 50 mol% SM-102 (LNP-38)

A certain amount of heptadecan-9-yl-8-(2-hydroxyethyl) (6-oxo-6-(undecyloxy)hexyl)amino)octoate (SM-102, Sinopeg), DSPC, cholesterol and mPEG-DMG-2k were weighed at a molar ratio of 50:10:38.5:1.5 and a lipid ethanol solution with a total lipid concentration of 23mg/mL was prepared. HPV mRNA (SEQ ID NO.2) was diluted with pH4.0 malic acid buffer and the mass ratio of lipids to HPV mRNA was 20:1. The foregoing lipid ethanol solution and the HPV mRNA aqueous solution were mixed at a volume ratio of 1:3 on a microfluidic device (Micro & Nano, INano L) at 50°C and a flow rate of 12mL/min, and the obtained LNP was dialyzed with pH7.4 PBS for 24h, to obtain the ultimate LNP-38 preparation.

### Preparation Example 39: Preparation of lipid nanoparticles containing 43.3 mol% compound 1 (LNP-39)

A certain amount of compound 1, DSPC, cholesterol and mPEG-DMG-2k were weighed at a molar ratio of 43.3:8.7:46.5:1.5 and a lipid ethanol solution with a total lipid concentration of 23mg/mL was prepared. HPV mRNA (SEQ ID NO.2) was diluted with pH4.0 malic acid buffer and the mass ratio of lipids to HPV mRNA was 20:1. The foregoing lipid ethanol solution and the HPV mRNA aqueous solution were mixed at a volume ratio of 1:3 on a microfluidic device (Micro & Nano, INano L) at 50°C and a flow rate of 12mL/min, and the obtained LNP was dialyzed with pH7.4 PBS for 24h, to obtain the ultimate LNP-39 preparation.

### Test examples

### Test example 1: Pharmaceutical characterization of different lipid nanoparticles mRNA-LNP made from different cationic lipid compounds

The particle size and PDI of mRNA-LNP were characterized by the dynamic light scattering method using a particle size meter (Malvern Zetasizer Nano-ZS). The encapsulation rate of mRNA (above SEQ ID NO.1 and SEQ ID NO.2) was determined using a Ribogreen RNA quantitative assay kit (Thermo Fisher) and ELIASA (Molecular adevices, SpectraMax i3x) at an excitation wavelength of 480 nm and an emission wavelength of 525 nm. The pKa of lipid nanoparticles was determined by 2-(p-toluidine)-6-naphthalenesulfonic acid (TNS) dye binding method using ELIASA (Molecular adevices, SpectraMax i3x) at an excitation wavelength of 325 nm and an emission wavelength of 435 nm. The pharmaceutical characterization is as shown in Table 1 and Table 2.

**Table 1. Particle size, PDI, Zeta potential, encapsulation rate and pKa results of mRNA-LNPs made from different cationic lipids**

| Cationic lipid | Sample No. | Proportion of cationic lipid (mol%) | Proportion of cholesterol (mol%) | Particle size (nm) | PDI | Zeta potential (mV) | Encapsulation rate (%) | pKa |
|---|---|---|---|---|---|---|---|---|
| Compound 1 | LNP-1 | 25.0 | 30.0 | 82.72 | 0.180 | -3.7 | 92.54 | 6.1 |
| | LNP-2 | 50.0 | 38.5 | 99.81 | 0.127 | -9.5 | 81.61 | 6.1 |
| Compound 2 | LNP-3 | 25.0 | 30.0 | 68.62 | 0.247 | -2.2 | 95.28 | 6.2 |
| | LNP-4 | 31.5 | 56.0 | 90.78 | 0.214 | -6.6 | 93.80 | 6.3 |
| | LNP-5 | 43.3 | 46.5 | 163.50 | 0.140 | -8.4 | 89.92 | |
| | LNP-6 | 46.3 | 42.7 | 172.70 | 0.139 | -6.8 | 88.27 | |
| | LNP-7 | 50.0 | 30.0 | 103.50 | 0.124 | -6.8 | 84.20 | |
| | LNP-8 | 50.0 | 38.5 | 142.80 | 0.136 | -4.2 | 88.97 | 6.1 |
| | LNP-9 | 57.1 | 34.3 | 170.05 | 0.097 | -8.5 | 84.89 | |
| MC3 | LNP-10 | 50.0 | 38.5 | 105.80 | 0.162 | -1.5 | 96.40 | 6.3 |
| Compound 9 | LNP-19 | 25.0 | 30.0 | 83.57 | 0.246 | 6.8 | 93.26 | 7.4 |
| Compound 10 | LNP-20 | 25.0 | 30.0 | 98.76 | 0.233 | 7.9 | 91.67 | 6.5 |
| Compound 11 | LNP-21 | 25.0 | 30.0 | 98.17 | 0.162 | 0.0 | 54.47 | |
| Compound 17 | LNP-35 | 50.0 | 38.5 | 83.90 | 0.230 | -1.2 | 98.98 | 6.9 |
| Compound 18 | LNP-36 | 50.0 | 38.5 | 77.36 | 0.168 | -1.3 | 99.15 | 7.1 |
| Compound 1 | LNP-37 | 36.5 | 46.0 | 57.10 | 0.141 | -9.7 | 97.03 | |
| SM-102 | LNP-38 | 50.0 | 38.5 | 75.72 | 0.101 | -5.1 | 97.04 | |
| Compound 1 | LNP-39 | 43.3 | 46.5 | 55.58 | 0.162 | -14.4 | 96.86 | |

**Table 2. Particle size, PDI, Zeta potential and encapsulation rate results of mRNA-LNPs made from different cationic lipids**

| Cationic lipid | Sample No. | Proportion of cationic lipid (mol%) | Particle size (nm) | PDI | Zeta potential (mV) | Encapsulation rate (%) |
|---|---|---|---|---|---|---|
| Compound 3 | LNP-11 | 25.0 | 145.70 | 0.212 | 17.5 | 97.29 |
| DOTAP | LNP-12 | 25.0 | 84.44 | 0.081 | 26.2 | 97.38 |
| Compound 4 | LNP-13 | 25.0 | 59.10 | 0.172 | 18.9 | 96.87 |
| Compound 5 | LNP-14 | 25.0 | 88.50 | 0.153 | 20.7 | 99.28 |
| Compound 6 | LNP-15 | 25.0 | 105.10 | 0.123 | 19.4 | 99.38 |
| Compound 6 | LNP-16 | 25.0 | 120.70 | 0.419 | 21.3 | 97.42 |
| Compound 7 | LNP-17 | 25.0 | 87.60 | 0.143 | 25.0 | 96.66 |
| Compound 8 | LNP-18 | 25.0 | 115.00 | 0.063 | 24.9 | 99.80 |

The results of Table 1 and Table 2 show that by adjusting the prescription ratio and process parameters, such as adjusting the proportions of cationic lipid and cholesterol, all compounds can be made into LNPs with small PDI, uniform particle size and high encapsulation rate.

### Test example 2: Experiment of cell transfection of different lipid nanoparticles made from different cationic lipid compounds

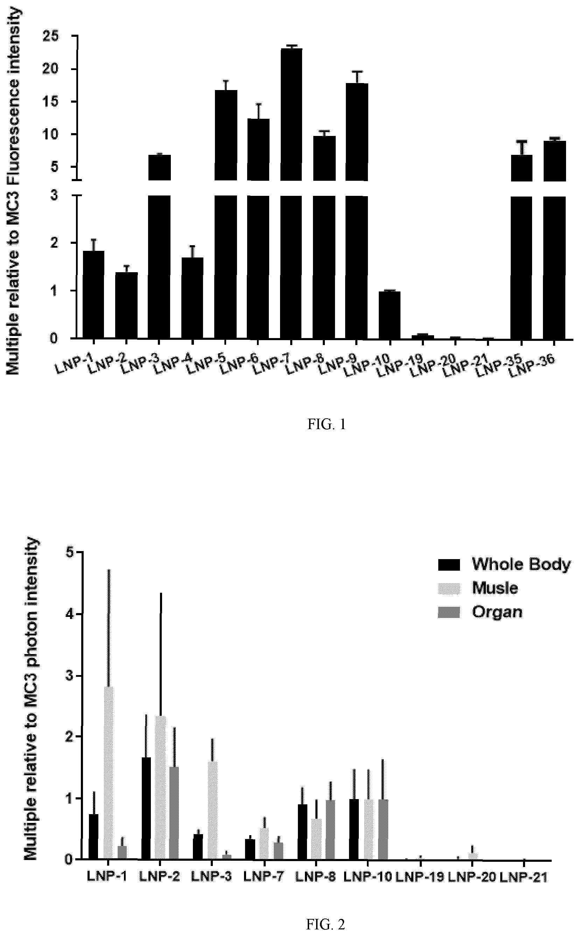

The well-grown Hela cells (BNCC) in the logarithmic growth phase were digested and re-suspended, then uniformly inoculated into 96-well cell culture plates with a cell density of 2×10⁴ cells/well, and incubated overnight in a cell incubator (Thermo Fisher, 1379) at 37 °C and 5% CO₂. 10 µL of sample diluents at different concentrations were added to 96-well cell culture plates so that each well contained 100 ng of FFluc mRNA (SEQ ID NO.1), and each sample was provided with 4 wells. The cells were incubated in a cell incubator for 24 h, then 60 µL of lysis solution containing D-fluorescein potassium salt (Vazyme, DD1201-02) was added to each well to lyse the cells and release the luciferase, and the liquid in the wells was blowed, beated and mixed, incubated at room temperature for 6 min, then transferred to white opaque bottom plates, and fluorescence intensity was measured by ELIASA. The results are as shown in the following Table 3, Table 4 and FIG. 1.

**Table 3. Results of transfection efficiency of lipid nanoparticles made from different cationic lipids**

| Cationic lipid | Sample No. | Proportion of cationic lipid (mol%) | Proportion of cholesterol (mol%) | Fluorescence intensity | Multiple relative to the strength of MC3 |
|---|---|---|---|---|---|
| Compound 1 | LNP-1 | 25.0 | 30.0 | 5.14E+05 | 1.83 |
| | LNP-2 | 50.0 | 38.5 | 3.92E+05 | 1.39 |
| Compound 2 | LNP-3 | 25.0 | 30.0 | 1.96E+06 | 6.96 |
| | LNP-4 | 31.5 | 56.0 | 4.79E+05 | 1.70 |
| | LNP-5 | 43.3 | 46.5 | 4.74E+06 | 16.84 |
| | LNP-6 | 46.3 | 42.7 | 3.50E+06 | 12.42 |
| | LNP-7 | 50.0 | 30.0 | 6.54E+06 | 23.23 |
| | LNP-8 | 50.0 | 38.5 | 2.77E+06 | 9.85 |
| | LNP-9 | 57.1 | 34.3 | 5.05E+06 | 17.95 |
| MC3 | LNP-10 | 50.0 | 38.5 | 2.81E+05 | 1.00 |
| Compound 9 | LNP-19 | 25.0 | 30.0 | 2.55E+04 | 0.09 |
| Compound 10 | LNP-20 | 25.0 | 30.0 | 8.83E+03 | 0.03 |
| Compound 11 | LNP-21 | 25.0 | 30.0 | 6.06E+03 | 0.02 |
| Compound 17 | LNP-35 | 50.0 | 38.5 | 1.99E+06 | 7.08 |
| Compound 18 | LNP-36 | 50.0 | 38.5 | 2.58E+06 | 9.18 |

The results in Table 3 and FIG. 1 show that compared with the existing commercially available cationic lipid MC3, the lipid nanoparticles made from ionizable cationic lipid material in different proportions in the present invention all show significantly higher in vitro FFluc mRNA (SEQ ID NO.1) transfection effect in Hela cells. For example, the in vitro FFluc mRNA transfection effect of LNP-7 in which the molar ratio of cationic lipid compound 2 is 50% is 23.23 times that of MC3 LNP-10; the in vitro FFluc mRNA transfection effect of LNP-9 in which the molar ratio of cationic lipid compound 2 is 57.1% is 17.95 times that of MC3 LNP-10; the in vitro FFluc mRNA transfection effect of LNP-5 in which the molar ratio of cationic lipid compound 2 is 43.3% is 16.84 times that of MC3 LNP-10.

**Table 4. Results of transfection efficiency of lipid nanoparticles made from different cationic lipids**

| Cationic lipid | Sample No. | Fluorescence intensity | Multiple relative to DOTAP fluorescence intensity |
|---|---|---|---|
| Compound 3 | LNP-11 | 2.95E+05 | 47.67 |
| DOTAP | LNP-12 | 6.20E+03 | 1.00 |
| Compound 4 | LNP-13 | 3.10E+03 | 0.50 |
| Compound 5 | LNP-14 | 7.77E+02 | 0.13 |
| Compound 6 | LNP-15 | 4.82E+04 | 7.77 |
| Compound 6 | LNP-16 | 7.67E+05 | 123.71 |
| Compound 7 | LNP-17 | 1.16E+04 | 1.87 |
| Compound 8 | LNP-18 | 1.18E+03 | 0.19 |

The results in Table 4 show that compared with the existing commercially available cationic lipid DOTAP, LNP-11 made from cationic lipid compound 3 in the present invention shows significantly higher in vitro FFluc mRNA transfection effect in Hela cells, which is 47.67 times that of DOTAP LNP-12. In addition, the transfection effect of LNP-16 made from cationic lipid compound 6 is 123.71 times that of DOTAP LNP-12.

### Test example 3: Experiment of animal transfection of different lipid nanoparticles made from different cationic lipid compounds (24h)

Balb/c mice (available from Vital River Laboratory Animal Technology Co., Ltd., male, 6-8 weeks old, 20-24 g) were randomized into groups, 5 mice per group. The mice were injected the prepared lipid nanoparticles into the leg muscle at a dose of 0.1 mg/kg (calculated as to FFluc mRNA (SEQ ID NO.1)) and observed by in vivo imaging after 24 h. The injection sites on the bellies and legs of Balb/c mice were depilated, and after anesthesia, the animals were injected D-luciferin potassium salt intraperitoneally, and were observed and photographed in an IVIS living body imager (PerkinElmer, Series III), wherein the observation time of living body images was within 15 min after the injection of substrate. The results are as shown in Table 5, Table 6 and FIG. 2-5.

**Table 5. Results of animal transfection efficiency of lipid nanoparticles made from different cationic lipids**

| Cationic lipid | Sample No. | Proportion of cationic lipid (mol%) | Proportion of cholesterol (mol%) | Multiple of total photon intensity in the supine position relative to MC3 | Multiple of local photon intensity in the supine position relative to MC3 | Multiple of photon intensity of the viscera in the supine position relative to MC3 |
|---|---|---|---|---|---|---|
| Compound 1 | LNP-1 | 25.0 | 30.0 | 0.74 | 2.82 | 0.23 |
| | LNP-2 | 50.0 | 38.5 | 1.67 | 2.35 | 1.51 |
| Compound 2 | LNP-3 | 25.0 | 30.0 | 0.42 | 1.61 | 0.10 |
| | LNP-7 | 50.0 | 30.0 | 0.35 | 0.53 | 0.29 |
| | LNP-8 | 50.0 | 38.5 | 0.92 | 0.68 | 0.98 |
| MC3 | LNP-10 | 50.0 | 38.5 | 1.00 | 1.00 | 1.00 |
| Compound 9 | LNP-19 | 25.0 | 30.0 | 0.01 | 0.05 | 0.00 |
| Compound 10 | LNP-20 | 25.0 | 30.0 | 0.03 | 0.13 | 0.00 |
| Compound 11 | LNP-21 | 25.0 | 30.0 | 0.00 | 0.02 | 0.00 |

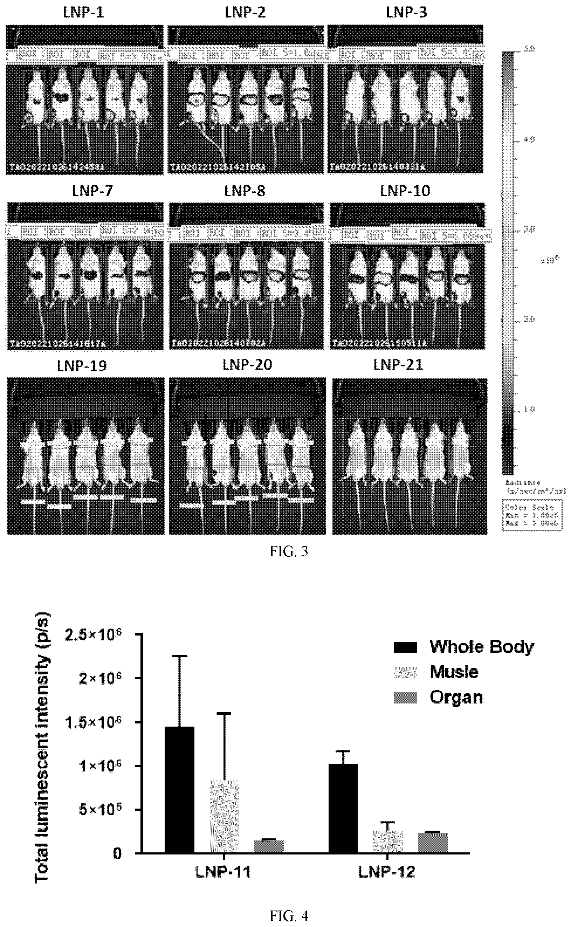

The results in Table 5, FIG. 2 and FIG. 3 show that compared with the existing commercially available cationic lipid MC3, the LNPs made from ionizable cationic lipid materials compound 1 and compound 2 provided by the present invention all have significantly higher transfection intensity at the injection site - thigh muscles than that of MC3 LNP. For example, the photon intensity of LNP-1 made from compound 1 at the injection site is 2.820 times that of MC3 LNP-10; the photon intensity of LNP-3 made from compound 2 at the injection site is 1.607 times that of MC3 LNP. In addition, the in vivo FFluc mRNA transfection efficiency of compound 2 at a high ratio of 50% is equivalent to that of MC3, and the in vivo transfection efficiency of compound 1 at a high ratio of 50% is 1.668 times that of MC3.

**Table 6. Results of animal transfection efficiency of lipid nanoparticles made from different cationic lipids**

| Sample No. | Photon intensity of the whole body in the supine position (p/s) | Photon intensity of the muscles in the supine position (p/s) | Photon intensity of the viscera in the supine position (p/s) | Multiple of photon intensity of the whole body in the supine position relative to DOTAP | Multiple of photon intensity of the muscles in the supine position relative to DOTAP | Multiple of photon intensity of the viscera in the supine position relative to DOTAP |
|---|---|---|---|---|---|---|
| LNP-11 | 1.46E+06 | 8.43E+05 | 1.54E+05 | 1.41 | 3.12 | 0.65 |
| LNP-12 | 1.04E+06 | 2.70E+05 | 2.39E+05 | 1.00 | 1.00 | 1.00 |

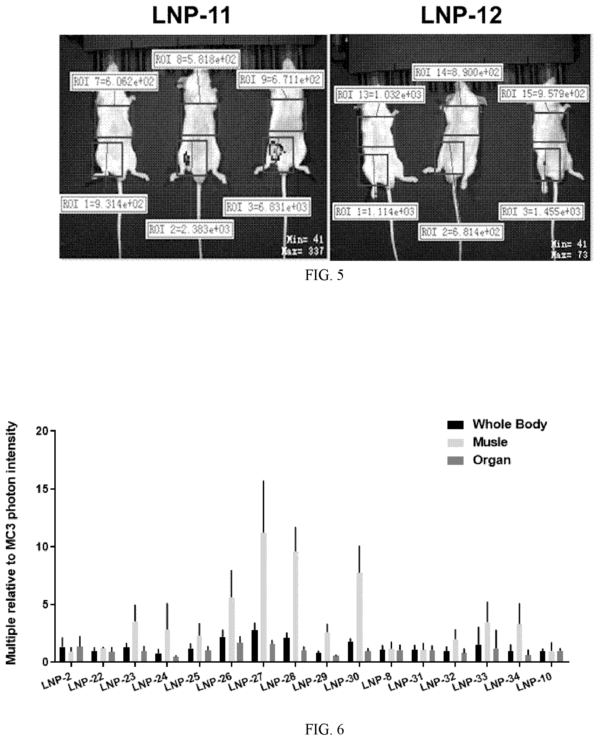

The results in Table 6, FIG. 4 and FIG. 5 show that the in vivo transfection efficiency of LNP-11 made from cationic lipid compound 3 provided by the present invention is significantly higher than that of the existing commercially available cationic lipid DOTAP LNP, the systemic luciferase expression of LNP-11 is 1.41 times that of DOTAP LNP-12, and the photon intensity at the injection site of LNP-11 is 3.12 times that of DOTAP LNP-12. This further indicates that LNP-11 made from compound 3 provided by the present invention is mainly expressed at the injection site, and the expression in the viscera is weak. Thus, compared with DOTAP LNP, LNP-11 made from compound 3 is expected to enhance the efficacy and reduce the toxicity.

### Test example 4: Animal transfection experiment of different lipid nanoparticles made from different cationic lipid compounds (12h)

Balb/c mice (available from Vital River Laboratory Animal Technology Co., Ltd., male, 6-8 weeks old, 20-24 g) were randomized into groups, 5 mice per group. The mice were injected the prepared lipid nanoparticles into the leg muscle at a dose of 0.1 mg/kg (calculated as to FFluc mRNA (SEQ ID NO.1)) and observed by in vivo imaging after 12 h. The injection sites on the bellies and legs of Balb/c mice were depilated, and after anesthesia, the animals were injected D-luciferin potassium salt intraperitoneally, and were observed and photographed in an IVIS living body imager (PerkinElmer, Series III), wherein the observation time of living body images was within 15 min after the injection of substrate. The results are as shown in Table 7, Table 8 and FIG. 6-9.

**Table 7. Results of animal transfection efficiency of lipid nanoparticles made from different cationic lipids**

| Cationic lipid | Sample No. | Proportion of cationic lipid (mol%) | Proportion of cholesterol (mol%) | Multiple of total photon intensity in the supine position relative to MC3 | Multiple of local photon intensity in the supine position relative to MC3 | Multiple of the photon intensity of the viscera in the supine position |
|---|---|---|---|---|---|---|
| | LNP-2 | 50.0 | 38.5 | 1.32 | 0.92 | 1.36 |
| | LNP-22 | 45.0 | 43.5 | 0.96 | 1.21 | 0.91 |
| | LNP-23 | 45.0 | 38.5 | 1.31 | 3.55 | 0.97 |
| | LNP-24 | 45.0 | 35.0 | 0.79 | 2.83 | 0.49 |
| Compound 1 | LNP-25 | 45.0 | 31.0 | 1.21 | 2.32 | 1.05 |
| | LNP-26 | 43.3 | 46.5 | 2.20 | 5.62 | 1.71 |
| | LNP-27 | 40.0 | 46.0 | 2.77 | 11.23 | 1.58 |
| | LNP-28 | 40.0 | 43.5 | 2.12 | 9.61 | 1.07 |
| | LNP-29 | 40.0 | 38.5 | 0.81 | 2.61 | 0.54 |
| | LNP-30 | 36.5 | 46.0 | 1.81 | 7.74 | 0.97 |
| Compound 2 | LNP-8 | 50.0 | 38.5 | 1.08 | 1.17 | 1.06 |
| | LNP-31 | 45.0 | 43.5 | 1.09 | 1.08 | 1.06 |
| | LNP-32 | 45.0 | 38.5 | 0.96 | 2.01 | 0.81 |
| | LNP-33 | 45.0 | 35.0 | 1.50 | 3.48 | 1.20 |
| | LNP-34 | 40.0 | 38.5 | 0.96 | 3.30 | 0.62 |
| MC3 | LNP-10 | 50.0 | 38.5 | 1.00 | 1.00 | 1.00 |

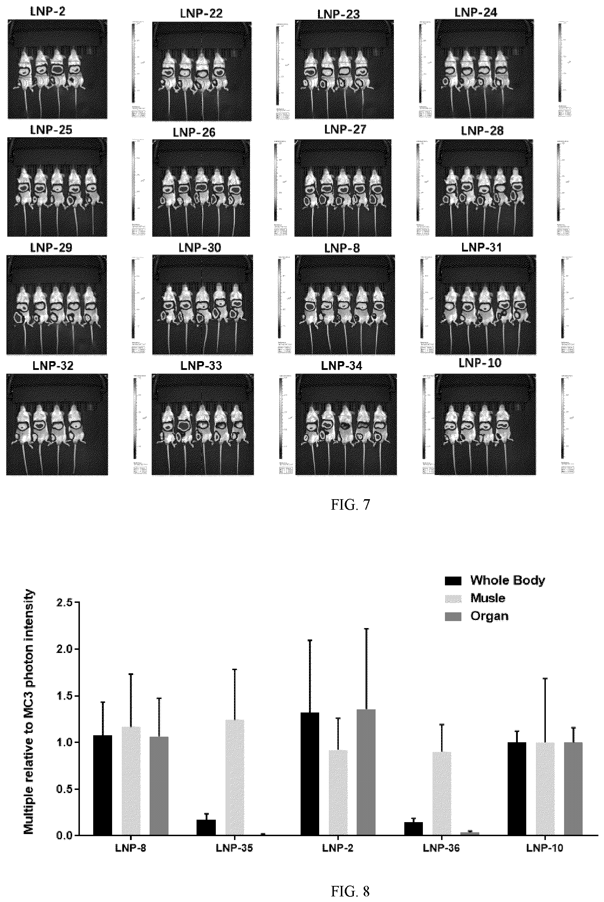

Compound 1 and compound 2 provided by the present invention have certain advantages over the existing commercially available cationic lipid MC3. Specifically speaking, the present invention explored the transfection characteristics 12h after administration of LNP prepared at different prescription ratios compared with the commercial prescription of MC3. As can be seen from Table 7, FIG. 6 and FIG. 7, the accumulations of all prescriptions in thigh muscles at the injection sites are not weaker than that of MC3 LNP, and those having specific prescriptions are significantly higher than that of MC3 LNP. For example, the photon intensity of LNP-27 made from compound 1 at the injection site is 11.23 times that of MC3 LNP; the photon intensity of LNP-33 made from compound 2 at the injection site is 3.48 times that of MC3 LNP. Further, the expressions of all prescriptions in the liver are close to that of MC3 LNP, indicating that they have similar liver toxic and side effect to MC3 LNP while maintaining a high transfection capacity.

**Table 8. Results of animal transfection efficiency of lipid nanoparticles made from different cationic lipids**

| Cationic lipid | Sample No. | Proportion of cationic lipid (mol%) | Proportion of cholesterol (mol%) | Multiple of total photon intensity in the supine position relative to MC3 | Multiple of local photon intensity in the supine position relative to MC3 | Multiple of photon intensity of the viscera in the supine position relative to MC3 |
|---|---|---|---|---|---|---|
| Compound 1 | LNP-2 | 50.0 | 38.5 | 1.32 | 0.92 | 1.36 |
| Compound 17 | LNP-35 | 50.0 | 38.5 | 0.17 | 1.24 | 0.02 |
| Compound 2 | LNP-8 | 50.0 | 38.5 | 1.08 | 1.17 | 1.06 |
| Compound 18 | LNP-36 | 50.0 | 38.5 | 0.14 | 0.90 | 0.04 |
| MC3 | LNP-10 | 50.0 | 38.5 | 1.00 | 1.00 | 1.00 |

As can be seen from Table 8, FIG. 8 and FIG. 9, 12h after administration, the accumulations of all the LNPs made from compound 1, compound 2, compound 17 and compound 18 provided by the present invention in thigh muscles at the injection sites are not weaker than that of MC3 LNP, and the LNPs made from compound 17 and compound 18 almost have no expression in the liver, indicating that the LNPs made from compound 17 and compound 18 completely avoid the toxic and side effect on the liver while achieving local transfection equivalent to MC3 LNP.

### Test example 5: Immunogenicity experiment of different lipid nanoparticles made from different cationic lipid compounds

C57BL/6 mice (available from Jiangsu Huachuang Sin Pharmaceutical Technology Co., Ltd, female, 6-8 weeks old, 20-22g) were randomized into three groups, 5 mice per group. The mice were administered saline, LNP-38 and LNP-39 to the inner thigh muscles, respectively, calculate according to HPV mRNA (SEQ ID NO.2) at a dose of 10µg/ mouse. The spleens of the mice were aseptically taken after one week of immunization, and mouse spleen cell suspension (1×10⁶ cells/mL) was prepared for future use.

The secretion of IFN-y was detected by an ELISPOT kit. The mouse spleen cell suspension at a volume of 100 µL/well was added into ELISPOT plates, and the stimulus was mixed with the corresponding cell suspension in the pore plates, wherein the one without stimulus was used as a negative control (NS), PMA as a positive control, and HPV 16 E7 Peptide pool as an experimental group (E7-PepLib). The ELISPOT plates was placed in a 37°C 5%CO₂ cell incubator (Thermo Fisher, 1379) and incubated overnight.

The ELISPOT plates were taken out, all liquid in the plates was discarded and the plates were washed, 100µL of prepared antibody solution was added to each well and incubated at room temperature for 2h. Then the liquid in the plates was discarded and the plates were washed, 100µL of prepared Streptavidin-ALP solution was added into each well and incubated at room temperature for 1h. Similarly, the liquid in the plates was discarded and the plates were washed, 100µL of chromogenic reagent was added to each well, rinsed with tap water 7 minutes after color development to terminate the color development, the above operation was repeated for three times and then the ELISPOT plates were air-dried. The spots were counted using a spot analyzer.

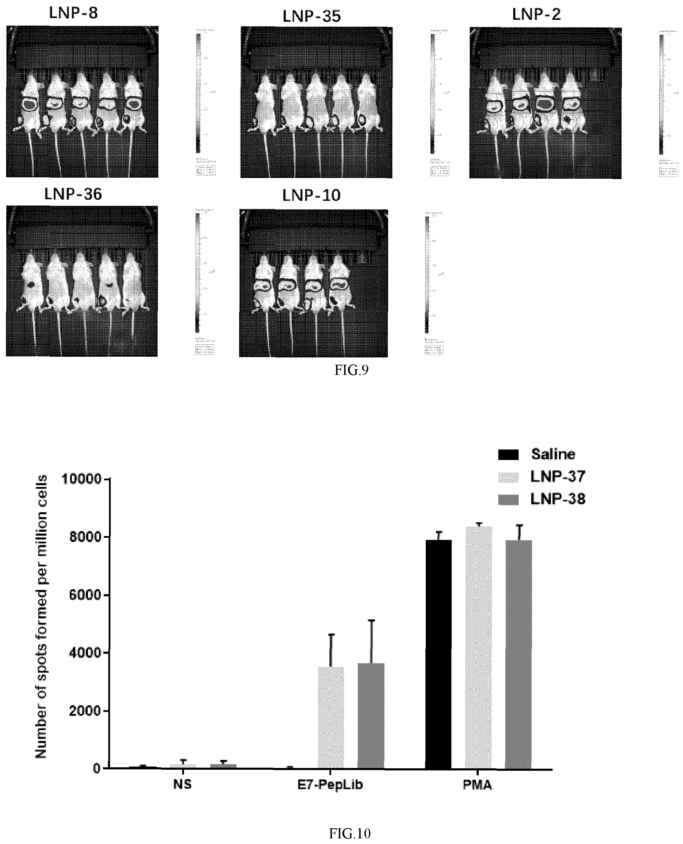

The immunogenicity results are shown in FIG. 10. There is no significant difference in mouse immunogenicity between LNP-37 made from compound 1 provided by the present invention and LNP-38 made from commercially available SM-102. This indicates that compound 1 can be used as an effective vector of mRNA vaccine to replace commercial cationic lipid SM-102, effectively stimulating the immune response of the body, and realizing effective prevention and treatment of target diseases.

### Test example 6: Antitumor experiment of different lipid nanoparticles made from different cationic lipid compounds

C3.43 cervical cancer cell suspension (1×10⁷ cells/mL, 200 µL, cell source: Keck Medicine of USC; the cells are of HPV16 transgenic cell line with C57BL/6 origin) was inoculated to site 3 and under the right armpit fat pad of C57BL/6 mice (available from Jiangsu Huachuang Sino Pharmaceutical Technology Co., Ltd., female, 6-8 weeks old, 20-22g), and when the tumor growed to 80-120 mm³ 10-12 days after inoculation, the tumor-bearing mice were randomized into 3 groups, 5 mice per group, and the mice were injected saline, LNP-38 and LNP-39 to the inner thigh muscles, respectively, and administered twice after 7 days, wherein the calculation was based on HPV mRNA (SEQ ID NO.2), and the dose was 10µg/mice. The tumor inhibition rate is as shown in Table 9.

**Table 9. Antitumor results of lipid nanoparticles made from different cationic lipids**

| Cationic lipid | Test substance | Tumor inhibition rate | | | | | | | |
|---|---|---|---|---|---|---|---|---|---|
| | | 0d | 3d | 7d | 9d | 14d | 17d | 21d | 24d |
| N/A | Saline | N/A | N/A | N/A | N/A | N/A | N/A | N/A | N/A |
| SM-102 | LNP-38 | 0% | 32% | 20% | 42% | 69% | 71% | 86% | 89% |
| Compound 1 | LNP-39 | 0% | 24% | 34% | 47% | 65% | 65% | 75% | 82% |

As can be seen from Table 9, after the two administrations, LNP-39 made from compound 1 provided by the present invention has a similar antitumor effect to that of LNP-38 made from commercially available cationic lipid SM-102. This indicates that compound 1 can be used as an effective vector of HPV mRNA vaccine to replace commercial cationic lipid SM-102, effectively stimulating the immune response of the body, and inhibiting HPV-associated tumor growth, which provides new possibilities for the future cancer treatment.

## Claims

1. A cationic lipid compound as shown in Formula (I) or a pharmaceutically acceptable salt thereof: 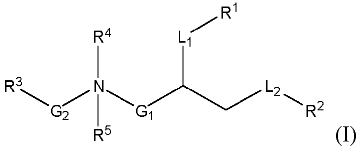 where,
R¹ and R² each independently are C₆-C₂₀ alkyl or C₆-C₂₀ alkenyl, or substituted C₂-C₅ alkyl, in which the substituent is -OC(=O)-, -C(=O)O-, -SC(=O)- or -C(=O)S- substituted by C₁₆-C₂₀ alkyl;
G₁ and G₂ each independently are C₁-C₄ alkylene;
L₁ and L₂ each independently are -OC(=O)-, -C(=O)O-, -SC(=O)- or -C(=O)S-;
R³ independently is a 5-6 membered saturated heterocyclic group containing 1 or 2 heterocyclic atoms independently selected from N, O and S and optionally substituted by R⁶;
R⁴ independently is C₁-C₄ alkyl, -OH or -SH, of which alkyl is optionally substituted by -OH or -SH;
R⁵ independently is absent or C₁-C₄ alkyl;
R⁶ independently is C₁-C₆ alkyl, which is optionally substituted by -OH or -SH.

2. The lipid compound according to claim 1, wherein R¹ and R² each independently are C₆-C₂₀ alkyl or C₆-C₂₀ alkenyl, preferably C₁₀-C₂₀ alkyl or C₁₀-C₂₀ alkenyl, more preferably C₁₅-C₂₀ alkyl or C₁₅-C₂₀ alkenyl.

3. The lipid compound according to claim 1 or 2, wherein R¹ and R² each independently are 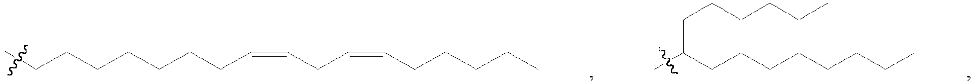 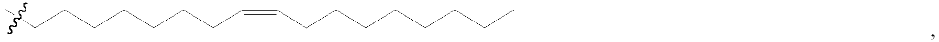 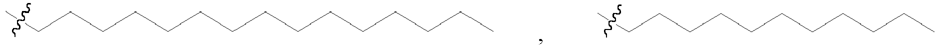 or

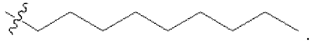

4. The lipid compound according to claim 1, wherein R¹ and R² each independently are substituted C₂-C₅ alkyl, preferably substituted C₄ alkyl, in which the substituent is -OC(=O)-, -C(=O)O-, -SC(=O)- or -C(=O)S-substituted by C₁₆-C₂₀ alkyl.

5. The lipid compound according to claim 1 or 4, wherein R¹ and R² each independently are

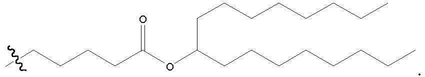

6. The lipid compound according to any one of claims 1 to 5, wherein G₁ independently is methylene, and G₂ independently is methylene or ethylene.

7. The lipid compound according to any one of claims 1 to 6, wherein R³ independently is pyrrolidinyl or piperazinyl, which is optionally substituted by R⁶, and R⁶ independently is methyl, ethyl, propyl or butyl, which is optionally substituted by -OH or -SH.

8. The lipid compound according to any one of claims 1 to 7, wherein R⁴ independently is methyl, hydroxymethyl, hydroxyethyl or -OH, and R⁵ is absent.

9. The lipid compound according to any one of claims 1 to 7, wherein R⁴ independently is methyl, and R⁵ independently is methyl.

10. The lipid compound according to any one of claims 1 to 9, which is an ionizable cationic lipid compound as shown in Formula (Ia) or a pharmaceutically acceptable salt thereof:

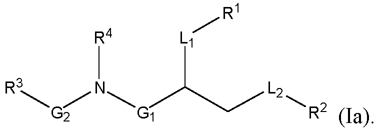

11. The lipid compound according to any one of claims 1 to 9, which is a non-ionized cationic lipid compound as shown in Formula (Ib) or a pharmaceutically acceptable salt thereof: 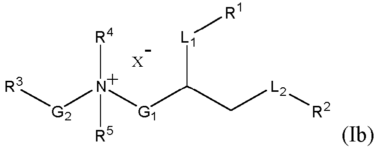 where, X is a chlorine, bromine or iodine atom.

12. The lipid compound according to any one of claims 1 to 11, which is a cationic lipid compound selected from any of the following compounds or a pharmaceutically acceptable salt thereof: 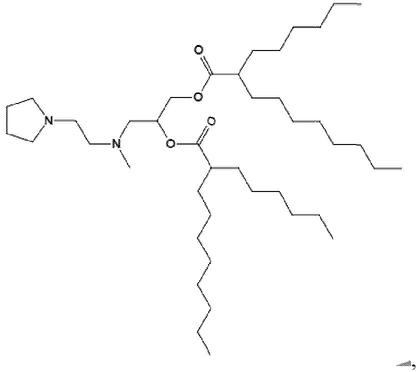 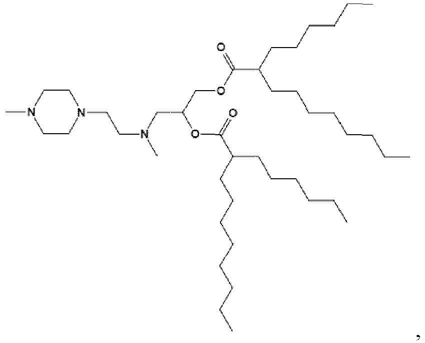 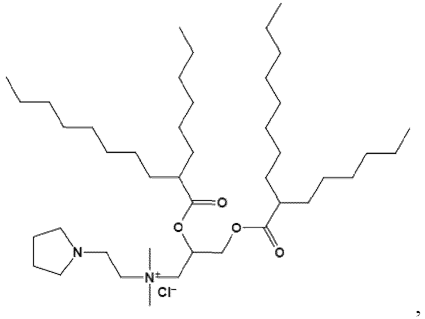 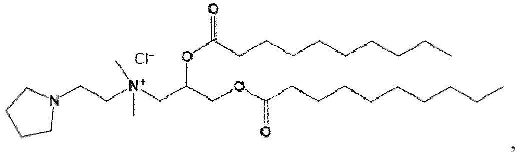 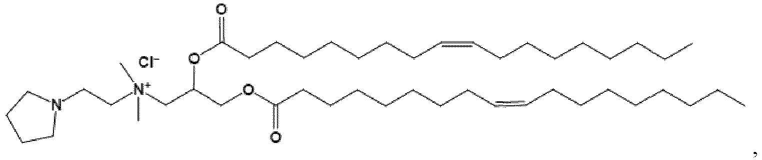 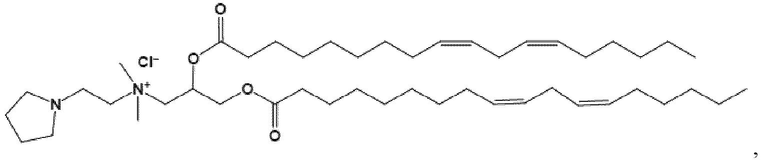 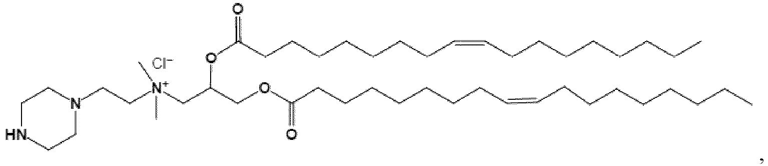 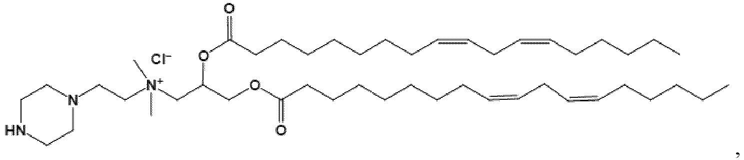 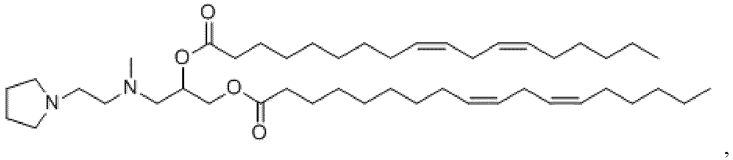 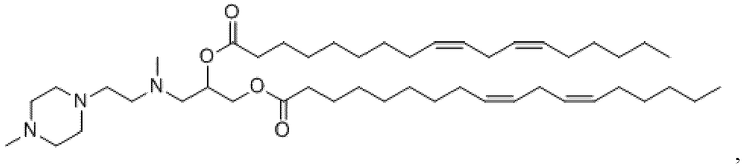 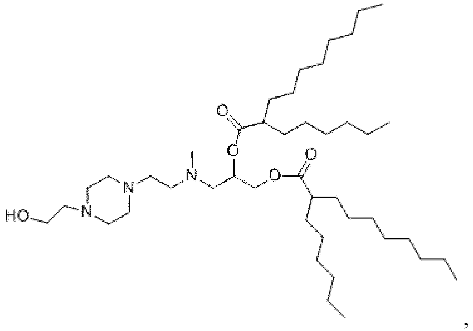 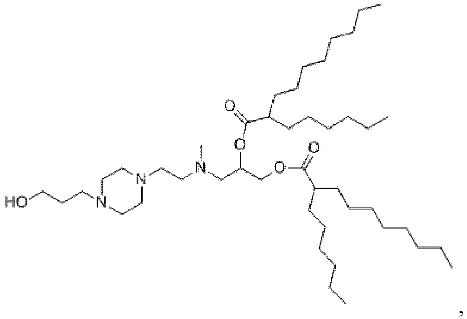 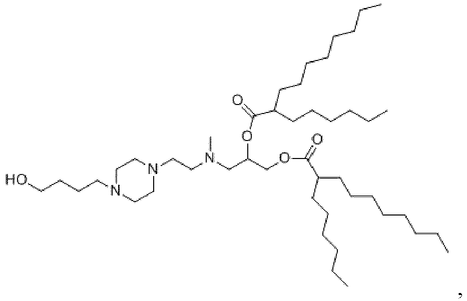 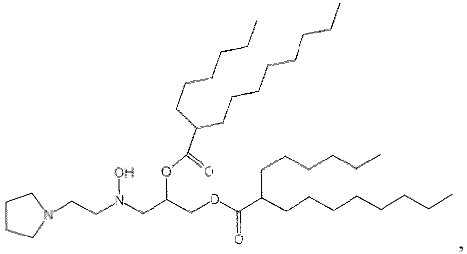 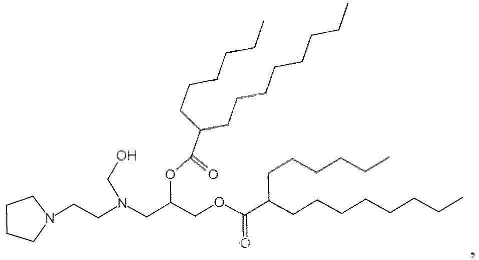 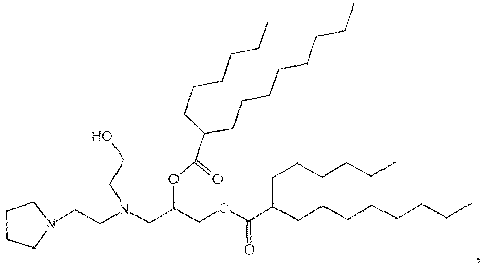 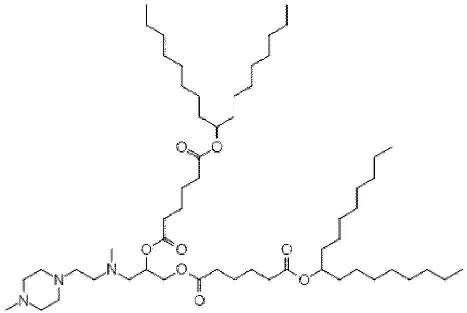 and

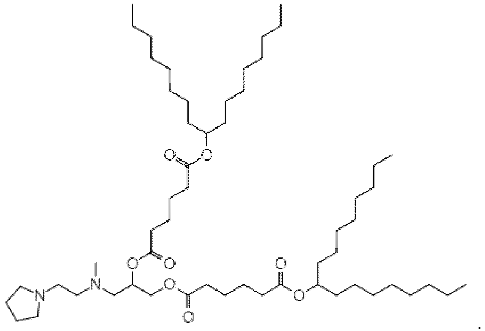

13. A lipid nanoparticle composition, containing the cationic lipid compound of any one of claims 1 to 12 or a pharmaceutically acceptable salt thereof.

14. The lipid nanoparticle composition according to claim 13, further containing neutral lipid, cholesterol and PEG lipid, wherein the neutral lipid is selected from DSPC, DOPC, DPPC, DOPG, DPPG, DOPE, POPC, POPE, DOPE-mal, DPPE, DMPE, DSPE, SOPE and 1,2-divaleryl-sn-glycerol-3-phosphoethanolamine (trans-DOPE), and the PEG lipid is selected from PEG-DMG, PEG-dipalmitoyl glycerol, PEG-DSPE, PEG-dilauryl glyceramide, PEG-dimyristyl glyceramide, PEG-dipalmitoyl glyceramide, PEG-distearoyl glyceramide, PEG-cholesterol(1-[8'-(cholest-5-ene-3[β]-oxo)formamido-3',6'-dioxazoctyl]carbamoyl-[ω]-methyl-poly(ethylen e glycol), PEG-DMB, mPEG-DMG-2k, PEG2k-DMG, PEG2k-DSPE, PEG2k-DSG, PEG2k-DMA and PEG2k-DSA.

15. The lipid nanoparticle composition according to any one of claims 13 to 14, wherein the molar percentage (mol %) of the cationic lipid compound to the total lipid components in the composition is 15-60%.

16. The lipid nanoparticle composition according to any one of claims 13 to 15, further containing a nucleic acid molecule, which is selected from mRNA, siRNA, antisense oligonucleotides (ASO), saRNA and miRNA.

17. A method for delivering nucleic acids into cells, comprising delivering the lipid nanoparticle composition according to any one of claims 13 to 16 into the cells, wherein the cells are mammalian cells, preferably human cells.

18. Use of the lipid nanoparticle composition in any one of claims 13 to 16 in the manufacture of a medicament for the treatment of diseases.
